(19) Europäisches Patentamt
European Patent Office
Office européen des brevets

(11) Publication number: 0 429 257 A2

# (12) EUROPEAN PATENT APPLICATION

(21) Application number: 90312489.9

(51) Int. Cl.[5]: C07D 403/06, A61K 31/415

(22) Date of filing: 16.11.90

(30) Priority: 17.11.89 GB 8926097
01.06.90 GB 9012276

(43) Date of publication of application:
29.05.91 Bulletin 91/22

(84) Designated Contracting States:
AT BE CH DE DK ES FR GB GR IT LI LU NL SE

(71) Applicant: GLAXO GROUP LIMITED
Clarges House 6-12 Clarges Street
London W1Y 8DH(GB)

(72) Inventor: Ross, Barry Clive
Glaxo Group Research Limited, Park Road
Ware, Hertfordshire, SG12 0DG(GB)
Inventor: Middlemiss, David
Glaxo Group Research Limited, Park Road
Ware, Hertfordshire, SG12 0DG(GB)
Inventor: Scopes, David Ian Carter
Glaxo Group Research Limited, Park Road
Ware, Hertfordshire, SG12 0DG(GB)
Inventor: Jack, Torquil Iain Maclean
Glaxo Group Research Limited, Park Road
Ware, Hertfordshire, SG12 0DG(GB)
Inventor: Cardwell, Kevin Stuart
Glaxo Group Research Limited, Park Road
Ware, Hertfordshire, SG12 0DG(GB)
Inventor: Dowle, Michael Dennis
Glaxo Group Research Limited, Park Road
Ware, Hertfordshire, SG12 0DG(GB)

(74) Representative: Marchant, James Ian et al
Elkington and Fife Beacon House 113
Kingsway
London WC2B 6PP(GB)

## (54) Indole derivatives.

(57) The invention provides compounds of the general formula (I):

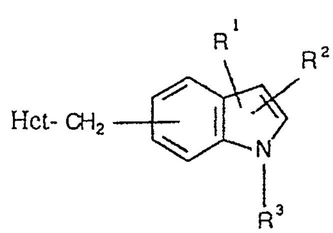

(I)

or a physiologically acceptable salt or a solvate (e.g. hydrates) thereof in which
$R^1$ represents a hydrogen atom or a halogen atom or a group selected from $C_{1-6}$ alkyl, $C_{2-6}$ alkenyl, fluoro$C_{1-6}$alkyl, -CHO, $-CO_2H$ or $-COR^4$;
$R^2$ represents a hydrogen atom or a halogen atom or the group Ar;
$R^3$ represents a hydrogen atom or a group selected from $C_{1-6}$ alkyl, $C_{3-6}$alkenyl, $C_{1-6}$alkoxy, $-COR^4$, $-SO_2R^4$ or the group Ar;
$R^4$ represents a group selected from $C_{1-6}$ alkyl, $C_{2-6}$ alkenyl, $C_{1-6}$ alkoxy or the group $-NR^{12}R^{13}$;
Ar represents the group

EP 0 429 257 A2

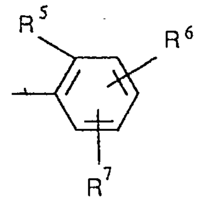

;

$R^5$ represents a group selected from $-CO_2H$, $-NHSO_2CF_3$ or a C-linked tetrazolyl group;
$R^6$ and $R^7$ which may be the same or different each independently represent a hydrogen atom or a halogen atom or a $C_{1-6}$alkyl group;
Het represents an N-linked imidazolyl group substituted at the 2-position by a $C_{1-6}$alkyl, $C_{2-6}$alkenyl or a $C_{1-6}$alkylthio group, the imidazolyl group optionally being substituted by one or two further substituents selected from a halogen atom or a group selected from cyano, nitro, $C_{1-6}$alkyl, $C_{2-6}$alkenyl, fluoro$C_{1-6}$alkyl, $-(CH_2)_mR^8$, $-(CH_2)_nCOR^9$, or $-(CH_2)_pNR^{10}COR^{11}$;
or Het represents an N-linked benzimidazolyl group substituted at the 2-position by a $C_{1-6}$alkyl, $C_{2-6}$alkenyl or a $C_{1-6}$alkylthio group;
$R^8$ represents a hydroxy or $C_{1-6}$alkoxy group;
$R^9$ represents a hydrogen atom or a group selected from hydroxy, $C_{1-6}$alkyl, $C_{1-6}$alkoxy, phenyl, phenoxy or the group $-NR^{12}R^{13}$;
$R^{10}$ represents a hydrogen atom or a $C_{1-6}$alkyl group;
$R^{11}$ represents a hydrogen atom or a group selected from $C_{1-6}$alkyl, $C_{1-6}$alkoxy, phenyl, phenoxy or the group $-NR^{12}R^{13}$;
$R^{12}$ and $R^{13}$ which may be the same or different each independently represent a hydrogen atom or a $C_{1-4}$alkyl group or $-NR^{12}R^{13}$ forms a saturated heterocyclic ring which has 5 or 6 ring members and may optionally contain in the ring one oxygen atom;
m represents an integer from 1 to 4;
n represents an integer from 0 to 4; and
p represents an integer from 1 to 4;
provided that one of $R^2$ and $R^3$ represents the group Ar, and that when $R^2$ represents the group Ar, $R^3$ represents a hydrogen atom or a group selected from $C_{1-6}$ alkyl, $C_{3-6}$ alkenyl, $C_{1-6}$alkoxy $-COR^4$ or $-SO_2R^4$, and that when $R^3$ represents the group Ar, $R^2$ represents a hydrogen atom or a halogen atom.

The compounds may be used in the treatment or prophylaxis of hypertension and diseases associated with cognitive disorders.

# INDOLE DERIVATIVES

This invention relates to indole derivatives, processes for their preparation and pharmaceutical compositions containing them. According to the invention we provide a compound of the general formula (I):

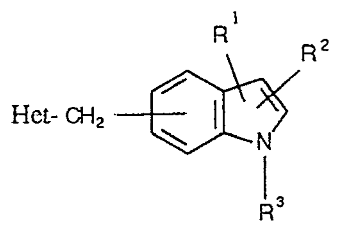

(I)

or a physiologically acceptable salt or a solvate (e.g. hydrates) thereof in which

$R^1$ represents a hydrogen atom or a halogen atom or a group selected from $C_{1-6}$ alkyl, $C_{2-6}$ alkenyl, fluoro$C_{1-6}$-alkyl, -CHO, $-CO_2H$ or $-COR^4$;

$R^2$ represents a hydrogen atom or a halogen atom or the group Ar;

$R^3$ represents a hydrogen atom or a group selected from $C_{1-6}$ alkyl, $C_{3-6}$alkenyl, $C_{1-6}$alkoxy, $-COR^4$, $-SO_2R^4$ or the group Ar;

$R^4$ represents a group selected from $C_{1-6}$ alkyl, $C_{2-6}$ alkenyl, $C_{1-6}$ alkoxy or the group $-NR^{12}R^{13}$;

Ar represents the group

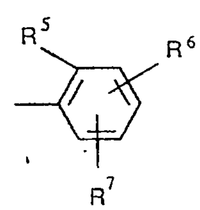

;

$R^5$ represents a group selected from $-CO_2H$, $-NHSO_2CF_3$ or a C-linked tetrazolyl group;

$R^6$ and $R^7$ which may be the same or different each independently represent a hydrogen atom or a halogen atom or a $C_{1-6}$alkyl group;

Het represents an N-linked imidazolyl group substituted at the 2-position by a $C_{1-6}$alkyl, $C_{2-6}$alkenyl or a $C_{1-6}$alkylthio group, the imidazolyl group optionally being substituted by one or two further substituents selected from a halogen atom or a group selected from cyano, nitro, $C_{1-6}$alkyl, $C_{2-6}$alkenyl, fluoro$C_{1-6}$alkyl, $-(CH_2)_mR^8$, $(CH_2)_nCOR^9$, or $(CH_2)_pNR^{10}COR^{11}$;

or Het represents an N-linked benzimidazolyl group substituted at the 2-position by a $C_{1-6}$alkyl, $C_{2-6}$alkenyl or a $C_{1-6}$alkylthio group;

$R^8$ represents a hydroxy or $C_{1-6}$alkoxy group;

$R^9$ represents a hydrogen atom or a group selected from hydroxy, $C_{1-6}$alkyl, $C_{1-6}$alkoxy, phenyl, phenoxy or the group $-NR^{12}R^{13}$;

$R^{10}$ represents a hydrogen atom or a cl6ajkyl group; $R^{11}$ represents a hydrogen atom or a group selectedfrom $C_{1-6}$alkyl, $C_{1-6}$alkoxy, phenyl, phenoxy or the group $-NR^{12}R^{13}$;

$R^{12}$ and $R^{13}$ which may be the same or different each independently represent a hydrogen atom or a $C_{1-4}$alkyl group or $-NR^{12}R^{13}$ forms a saturated heterocyclic ring which has 5 or 6 ring members and may optionally contain in the ring one oxygen atom;

m represents an integer from 1 to 4;

n represents an integer from 0 to 4; and

p represents an integer from 1 to 4;

provided that one of $R^2$ and $R^3$ represents the group Ar, and that when $R^2$ represents the group Ar, $R^3$ represents a hydrogen atom or a group selected from $C_{1-6}$ alkyl, $C_{36}$ alkenyl, $C_{1-6}$alkoxy $-COR^4$ or $-SO_2R^4$, and that when $R^3$ represents the group Ar, $R^2$ represents a hydrogen or a halogen atom.

Where optical isomers may exist formula (I) is intended to cover all enantiomers, diastereoisomers and

mixtures thereof including racemates. Compounds containing one or two double bonds may exist in the cis or trans configuration.

The invention also includes within its scope the solvates, especially the hydrates of compounds of general formula (I) or a physiologically acceptable salt thereof.

Within the above definition the term 'alkyl' or 'alkoxy' as a group or part of a group means that the group is straight or branched. The term 'alkenyl' as a group or part of a group means that the group is straight or branched and contains at least one carbon-carbon double bond. Furthermore, when $R^3$ represents a $C_{3-6}$ alkenyl group, the carbon-carbon double bond should not be in conjugation with the indole nitrogen.

The term 'halogen' means a fluorine, chlorine, bromine or iodine atom.

The term 'fluoro$C_{1-6}$alkyl' means an alkyl group in which one or more hydrogen atoms have been replaced by a fluorine atom, for example, $-CH_2CF_3$ or trifluoromethyl.

Within the above definition when $-NR^{12}R^{13}$ represents a saturated heterocyclic ring, this contains 5 or 6 ring members, one of which may be an oxygen atom. Suitable heterocyclic groups are a pyrrolidino, piperidino or morpholino group.

A preferred class of compounds of general formula (I) is that wherein the group Het is substituted at the 2-position by a $C_{2-5}$ alkyl, and in particular a $C_{3-5}$alkyl group, or $C_{3-5}$alkenyl group. Particularly preferred are those compounds wherein the 2-position substituent is an n-butyl or but-1-enyl group.

Another preferred class of compounds of general formula (I) is that wherein the group Het is optionally substituted by one or two further substituents selected from a halogen atom or a group selected from $C_{1-6}$ alkyl, $-(CH_2)_mR^8$ or $-(CH_2)_nCOR^9$. Particularly preferred are the compounds wherein Het is substituted by a halogen atom. Also preferred, are those compounds wherein Het is substituted by a group selected from $-(CH_2)_mR^8$ or $-(CH_2)_nCOR^9$, especially wherein $R^8$ represents hydroxy or methoxy and $R^9$ represents hydrogen, hydroxy, methoxy or ethoxy, especially hydrogen, hydroxy or methoxy and m is 1 or 2 and n is 0, 1 or 2. In particular, the substituents may represent a chlorine atom or a group selected from $-CH_2OH$, $-CHO$, $-CO_2H$, $-CH_2OCH_3$ or $-CO_2CH_2CH_3$.

Conveniently, in the compounds of general formula (I), $R^3$ may be a hydrogen atom.

A yet further preferred class of compounds of general formula (I) is that wherein $R^1$ is attached at the 3-position on the indole ring. Also preferred are those compounds wherein $R^1$ represents a hydrogen atom or a halogen (especially bromine) atom or a $C_{1-3}$alkyl group.

Another preferred class of compounds of general formula (I) is that wherein $R^2$ represents the group Ar, and in particular, where $R^1$ additionally represents a halogen atom and $R^3$ is a hydrogen atom.

Conveniently, in the compounds of general formula (I), the group Het-$CH_2$- is attached at the 5-position on the indole ring.

Also conveniently, in the compounds of general formula (I), $R^5$ may be the group $-CO_2H$ or a C-linked tetrazolyl group.

Still conveniently, in the compounds of general formula (I), $R^6$ and $R^7$ may each independently represent a hydrogen atom.

A particularly preferred class of compound of general formula (I) are those wherein

$R^1$ represents a hydrogen atom or a halogen atom;

$R^2$ represents a halogen atom or the group Ar;

$R^3$ represents a hydrogen atom, a $C_{1-6}$alkyl group or the group Ar;

Ar represents the group

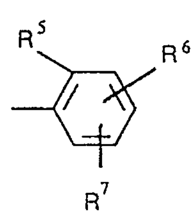

;

$R^5$ represents a group selected from $-CO_2H$, $-NHSO_2CF_3$ or a C-linked tetrazolyl group;

$R^6$ and $R^7$ each independently represent hydrogen atoms;

Het represents an N-linked imidazolyl group substituted at the 2-position by a $C_{1-6}$alkyl group, the imidazolyl group optionally being substituted by one or two further substituents selected from a halogen atom or a group selected from $-(CH_2)_mR^8$ or $-(CH_2)_nCOR^9$;

or Het represents an N-linked benzimidazolyl group substituted at the 2-position by a $C_{1-6}$alkyl group;
$R^8$ represents a hydroxy group;
$R^9$ represents a hydroxy group;
m represents an integer from 1 to 4; and
n represents an integer from 0 to 4;
provided that one of $R^2$ and $R^3$ represents the group Ar, and that when $R^2$ represents the group Ar, $R^3$ represents a hydrogen atom or a $C_{1-6}$alkyl group, and that when $R^3$ represents the group Ar, $R^2$ represents a halogen atom;
or a physiologically acceptable salt or solvate thereof.

Particularly preferred compounds of the invention include:
1-[[3-bromo-2-[2-(1H-tetrazol-5-yl)phenyl]-1H-indol-5-yl]methyl]-2-butyl-4-chloro-1H-imidazole-5-carboxylic acid;
1-[[3-bromo-2-[2-[[(trifluoromethyl)sulphonyl]amino]phenyl]-1H-indol-5-yl]methyl]-2-butyl-4-chloro-1H-imidazole-5-carboxylic acid;
or a physiologically acceptable salt or solvate thereof.

The physiologically acceptable acid addition salts of the compounds of formula (I) may be derived from inorganic or organic acids. Examples of such salts include hydrochlorides, hydrobromides, sulphates, phosphates, benzoates, naphthoates, hydroxynaphthoates, p-toluenesulphonates, methanesulphonates, sulphamates, ascorbates, oxalates, tartrates, citrates, maleates, salicylates, fumarates, succinates, lactates, glutarates, glutaconates, acetates, trifluoroacetates or tricarballylates.

The compounds may also form salts with suitable bases. Examples of such salts are alkali metal (e.g. sodium or potassium), alkaline earth metal (e.g. calcium or magnesium), ammonium and substituted ammonium (e.g. dimethylammonium, triethylammonium, 2-hydroxyethyldimethylammonium, piperazinium, N,N-dimethyl-piperazinium, piperidinium, ethylenediammonium and choline).

It will be appreciated that, for pharmaceutical use, the salts referred to above will be physiologically acceptable, but other salts may find use, for example, in the preparation of the compounds of formula (I) and the physiologically acceptable salts thereof.

It will be further appreciated that the compounds of general formula (I) may be chemically modified in the form of compounds which in vivo (for example, by enzymic attack) will liberate the parent compounds of general formula (I). Such prodrugs may be, for example, physiologically acceptable metabolically labile ester derivatives. These may be formed by esterification, for example of any of the carboxylic acid groups in the parent compound of general formula (I), with prior protection of any other reactive groups present in the molecule. Examples of such esters include lower alkane esters such as methyl or ethyl esters. In addition to the above ester derivatives the present invention includes within its scope compounds of general formula (I) in the form of other physiologically acceptable equivalents, i.e. physiologically acceptable compounds which like the metabolically labile esters are converted in vivo into the parent compounds of general formula (I).

According to a second aspect of the invention we provide a compound of formula (I) or a physiologically acceptable salt or a solvate thereof for use in therapy.

In particular, the compounds of the invention may be used in the treatment or prophylaxis of hypertension. They may also be used in the treatment or prophylaxis of cognitive disorders such as dementia (e.g. Alzheimer's disease) and other diseases such as renal failure, hyperaldosteronism, cardiac insufficiency, congestive heart failure, post-myocardial infarction, cerebrovascular disorders, glaucoma and disorders of intracellular homeostasis.

According to a further aspect of the invention we provide a compound of formula (I) or a physiologically acceptable salt or a solvate thereof for use in the treatment of the aforementioned diseases, especially hypertension.

According to another aspect of the invention we provide a compound of formula (I) or a physiologically acceptable salt or a solvate thereof for the manufacture of a therapeutic agent for the treatment of the aforementioned diseases, especially hypertension.

According to a further aspect of the invention we provide a method of treating the aforementioned diseases, especially hypertension, which method comprises administering an effective amount to a patient in need of such treatment of a compound of formula (I) or a physiologically acceptable salt or a solvate thereof.

It will be appreciated that the compounds of formula (I) or a physiologically acceptable salt or a solvate thereof may advantageously be used in conjunction with one or more other therapeutic agents, such as for example diuretics and/or different antihypertensive agents such as $\beta$-blockers, calcium channel blockers or ACE inhibitors. It is to be understood that such combination therapy constitutes a further aspect of the

present invention.

While it is possible that a compound of general formula (I) may be administered as the raw chemical it is preferable to present the active ingredient as a pharmaceutical formulation.

The compounds of formula (I) and their physiologically acceptable acid addition salts, solvates and metabolically labile esters may be formulated for administration in any convenient way, and the invention also includes within its scope pharmaceutical compositions comprising at least one compound of formula (I) or a physiologically acceptable salt, solvate or metabolically labile ester thereof adapted for use in human or veterinary medicine. Such compositions may be presented for use in conventional manner in admixture with one or more physiologically acceptable carriers or excipients. The carrier(s) must be 'acceptable' in the sense of being compatible with the other ingredients of the formulation and not deleterious to the recipient thereof.

Thus, the compounds according to the invention may be formulated for oral, buccal, parenteral or rectal administration or in a form suitable for administration by inhalation or insufflation. Oral administration is preferred.

Tablets and capsules for oral administration may contain conventional excipients such as binding agents, for example mucilage of starch or polyvinylpyrrolidone; fillers, for example, lactose, microcrystalline cellulose or maize-starch; lubricants, for example, magnesium stearate or stearic acid; disintegrants, for example, potato starch, croscarmellose sodium or sodium starch glycollate; or wetting agents such as sodium lauryl sulphate. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, for example, sorbitol syrup, methyl cellulose, glucose/sugar syrup or carboxymethyl cellulose; emulsifying agents, for example, sorbitan mono-oleate; non-aqueous vehicles (which may include edible oils), for example, propylene glycol or ethyl alcohol; and preservatives, for example, methyl or propyl p-hydroxybenzoates or sorbic acid. The compounds or their salts or esters may also be formulated as suppositories, e.g. containing conventional suppository bases such as cocoa butter or other glycerides. For buccal administration the composition may take the form of tablets or lozenges formulated in conventional manner.

It will be appreciated that both tablets and capsules may be manufactured in the form of sustained release formulations, such that they provide a controlled continuous release of the compounds according to the invention over a period of hours.

The compounds of formula (I) and their physiologically acceptable acid addition salts, solvates and metabolically labile esters may be formulated for parenteral administration by bolus injection or continuous infusion and may be presented in unit dose form in ampoules, or in multi-dose containers with an added preservative. The compositions may take such forms as suspensions, solutions, or emulsions in oily or aqueous vehicles, and may contain formulatory agents such as suspending, stabilising and/or dispersing agents. Alternatively the active ingredient may be in powder form for constitution with a suitable vehicle, e.g. sterile, pyrogen-free water, before use.

For administration by inhalation the compounds according to the invention are conveniently delivered in the form of an aerosol spray presentation from pressurised packs or a nebuliser, with the use of a suitable propellant, e.g. dichlorodifluoromethane, trichlorofluoromethane, dichlorotetrafluoroethane or other suitable gas. In the case of a pressurised aerosol the dosage unit may be determined by providing a valve to deliver a metered amount.

Alternatively, for administration by inhalation or insufflation, the compounds according to the invention may take the form of a dry powder composition, for example a powder mix of the compound and a suitable powder base such as lactose or starch. The powder composition may be presented in unit dosage form in, for example, capsules or cartridges of e.g. gelatin, or blister packs from which the powder may be administered with the aid of an inhaler or insufflator.

The pharmaceutical formulations according to the invention may also contain other active ingredients such as antimicrobial agents, or preservatives.

It will be appreciated that the amount of a compound of general formula (I) required for use in treatment will vary not only with the particular compound selected but also with the route of administration, the nature of the condition being treated and the age and condition of the patient and will ultimately be at the discretion of the attendant physician or veterinarian. In general, however, when the compositions comprise dosage units, each unit will preferably contain 5mg to 500mg, advantageously where the compounds are to be administered orally 25mg to 400mg of the active compound. The daily dosage as employed for adult human treatment will preferably range from 5mg to 3g, most preferably from 25mg to 1g which may be administered in 1 to 4 daily doses.

The compounds of the invention may be prepared by a number of processes as described below wherein the various groups are as defined for general formula (I) unless otherwise specified.

Thus, according to a further aspect of the present invention we provide a process (A) for preparing the compounds of general formula (I) which comprises treating the indole of general formula (II)

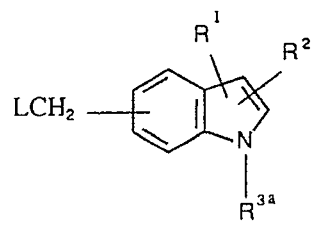

(II)

(wherein L is a leaving group, for example a halogen atom such as chlorine, bromine or iodine, or a hydrocarbylsulphonyloxy group such as methanesulphonyloxy, or p-toluenesulphonyloxy, $R^1$ and $R^2$ are as defined in general formula (I) and $R^{3a}$ is as defined for $R^3$ in general formula (I) or alternatively represents a nitrogen protecting group) with an imidazole of formula (III)

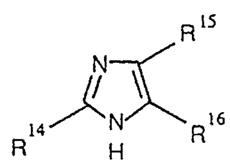

(III)

(wherein $R^{14}$ represents a group selected from $C_{1-6}$alkyl, $C_{2-6}$alkenyl or $C_{1-6}$alkylthio; $R^{15}$ and $R^{16}$ which may be the same or different each independently represent a hydrogen or halogen atom, or a group selected from cyano, nitro, $C_{1-6}$alkyl, $C_{2-6}$alkenyl, fluoro$C_{1-6}$alkyl, $(CH_2)_mR^8$, $-(CH_2)_nCOR^9$, $(CH_2)_pNR^{10}COR^{11}$ or when taken together with the carbon atoms to which $R^{15}$ and $R^{16}$ are attached, there is formed a fused phenyl ring; and $R^8$, $R^9$, $R^{10}$, $R^{11}$, m, n and p are as defined in general formula (I)) followed by the removal of any protecting groups where present, as described hereinafter.

The reaction is preferably effected under basic conditions, for example, in the presence of sodium hydride, potassium carbonate or sodium methoxide. The reaction is conveniently effected in a solvent such as acetonitrile or an ether e.g. tetrahydrofuran or dioxan, a ketone e.g. butanone or methyl isobutyl ketone, or a substituted amide e.g. dimethylformamide, at a temperature between 0°C and the reflux temperature of the solvent.

The intermediate indoles of general formula (II) and their acid addition salts are novel compounds and form a further aspect of the invention.

In another general process (B) a compound of general formula (I) may be obtained by deprotection of a protected intermediate of general formula (IV)

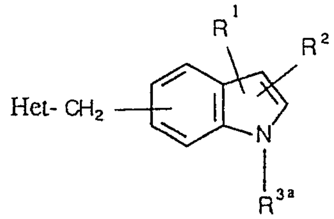

(IV)

(wherein $R^1$, $R^2$ and Het are as defined in general formula (I), $R^{3a}$ is as defined in general formula (II) and at least one reactive group is blocked by a protecting group, for example, when $R^{3a}$ represents a protecting group).

The protecting groups may be any conventional protecting groups, for example as described in "Protective Groups in Organic Synthesis" by Theodora Greene (John Wiley and Sons Inc., 1981). Examples of suitable nitrogen protecting groups such as that represented by $R^{3a}$ include acyl groups such as acetyl,

trichloroacetyl or trifluoroacetyl, carbamates such as methyl carbamate, t-butyl carbamate (t-BOC) or p-nitrobenzyl carbamate, silyl groups such as t-butyldimethylsilyl (TBDMS), or aryl sulphonyl groups such as phenylsulphonyl. Examples of carboxyl protecting groups include $C_{1-6}$ alkyl such as methyl or t-butyl, or $C_{7-10}$ aralkyl such as benzyl.

When $R^6$ is a tetrazole group, this may be protected with, for example, the trityl group -C(phenyl)$_3$, or a p-nitrobenzyl or 1-ethoxyethyl group.

Deprotection to yield the compound of general formula (I) may be effected using conventional techniques. Thus, for example, aralkyl groups may be cleaved by hydrogenolysis in a suitable organic solvent such as an alcohol, for example, ethanol in the presence of a noble metal catalyst such as palladium or platinum or an oxide thereof on a support such as charcoal, and conveniently at room temperature and pressure. When $R^{3a}$ is a carbamate such as t-BOC or an arylsulphonyl group, and optionally a carboxyl group is protected by an alkyl group, these may be cleaved by hydrolysis using a base such as an alkali metal hydroxide (e.g. sodium hydroxide or potassium hydroxide) in a suitable solvent (e.g. an aqueous alcohol such as methanol or ethanol) at any suitable temperature up to reflux. Deprotection of the tetrazole group when protected with a trityl group and removal of a TBDMS group may be effected by acid hydrolysis using trifluoroacetic acid or a mineral acid such as hydrochloric acid in a suitable solvent such as acetone conveniently at room temperature. Alternatively, when possible, deprotection of the tetrazolyl group can be effected by catalytic hydrogenation as previously described. Removal of a TBDMS group may also be effected by treatment with tetrabutylammoniumfluoride in dimethylformamide.

In another general process (C) a compound of general formula (I) in which the substituent $R^5$ in the group Ar represents a C-linked tetrazolyl group, may also be prepared from a compound of general formula (Ia)

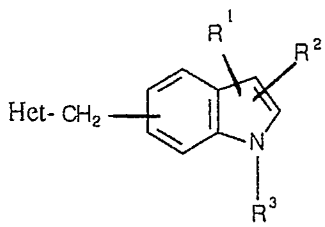

(Ia)

(wherein $R^1$, $R^2$, $R^3$ and Het are as defined in general formula (I) except that in the group Ar, $R^5$ represents a nitrile group) by reaction with a suitable azide such as sodium azide, ammonium azide (preferably prepared in situ from sodium azide and ammonium chloride) or tributyl tin azide. The reaction is conveniently effected in a solvent such as xylene or an ether, for example, dimethoxyethane or tetrahydrofuran at an elevated temperature, such as the reflux temperature of the solvent, for between 1 and 10 days. Where the azide is tributyl tin azide the reaction may conveniently be effected in the absence of a solvent at a temperature between room temperature and 180°C. Such a reaction leaves the tetrazolyl group protected with a tributyl tin group, which can readily be removed using aqueous base or acid. Where aqueous base is used to effect this deprotection, the compound may be treated with an aqueous acid to liberate the free acid.

The intermediate indoles of general formula (Ia) and their acid addition salts are novel compounds and form a further aspect of the invention.

In another general process (D), a compound of general formula (I) in which the substituent $R^5$ in the group Ar represents $-NHSO_2CF_3$, may also be prepared from a compound of general formula (Ib)

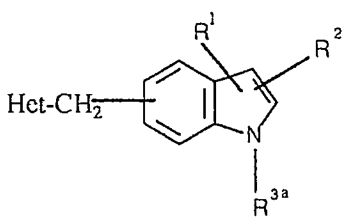

(1b)

wherein $R^1$, $R^2$, $R^3$ and Het are as defined in general formula (I) except that in the group Ar, $R^5$ represents

an amino groups) by reaction with trifluoromethanesulphonic anhydride in the presence of a base such as triethylamine, in a suitable solvent such as dichloromethane.

Compounds of general formula (Ib) may be prepared by processes analogous to those described herein commencing from a compound of formula (XIII) and a corresponding indole intermediate.

Alternatively, compounds of general formula (Ib) may be prepared by a Curtius rearrangement of a compound of formula (I) wherein $R^5$ in the group Ar is $-CO_2H$ (provided that this is the only carboxyl group in the molecule) using, for example, diphenylphosphorylazide in the, presence of a base such as triethylamine and in a solvent such as an alcohol (e.g. tert-butanol) to form a carbamate followed by deprotection of the amine in a conventional manner, for example by acid hydrolysis using hydrochloric acid in a solvent such as ethanol.

The intermediate compounds of general formula (Ib) and their acid additions salts are novel compounds and form a further aspect of the present invention.

In the processes (A), (B), (C) and (D) described above, the compounds of general formula (I) may be obtained in the form of a salt, conveniently in the form of a physiologically acceptable salt. Where desired, such salts may be converted into the corresponding free acids or free bases using conventional methods.

Physiologically acceptable salts of the compounds of general formula (I) may be prepared by reacting a compound of general formula (I) with an appropriate acid or base in the presence of a suitable solvent such as acetonitrile, acetone, chloroform, ethyl acetate or an alcohol, e.g. methanol, ethanol or isopropanol.

Physiologically acceptable salts may also be prepared from other salts, including other physiologically acceptable salts, of the compounds of general formula (I), using conventional methods.

The intermediate compounds of general formula (II) may be prepared from an indole of formula (V)

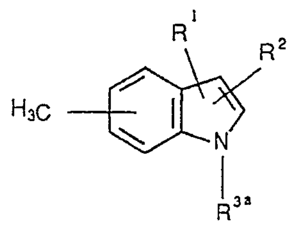

(V)

(wherein $R^{3a}$ is as defined in general formula (II)) using any suitable reagent well known in the art for converting the methyl on the 6-membered ring into the group $-CH_2L$ (wherein L is as defined in general formula (II)). Thus, for example, when L is a halogen atom, a compound of formula (V) can be converted into a compound of general formula (II) using N-chloro amides, tert-butyl hypochlorite or N-bromosuccinimide in the presence of a radical initiator such as azobisisobutyronitrile (AIBN) or dibenzoyl peroxide. Halogenation of the side chain may be catalysed by light, thus the reaction can be illuminated with a suitable artificial light source.

When $R^1$ is a halogen atom, L and $R^1$ can be the same and thus a one-step reaction with a halogenating agent can be effected.

Indoles of formula (V) wherein $R^2$ is Ar and is attached at the 2-position on the indole ring may be prepared by reaction of a compound of formula (VI)

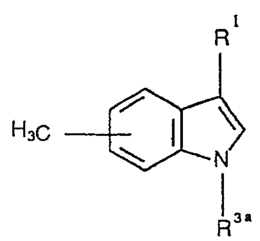

(VI)

with a compound of formula (VII)

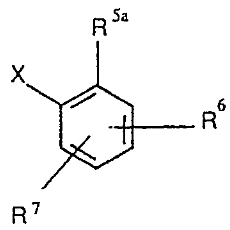

(VII)

(wherein X represents a bromine or iodine atom, $R^6$ and $R^7$ are as defined in general formula (I) and $R^{5a}$ is as defined for $R^5$ in general formula (I) or is a protected derivative thereof).

When $R^1$ in formula (VI) represents a hydrogen atom or a group selected from $C_{1-6}$alkyl or $C_{2-6}$alkenyl the compound of formula (VI) is first treated with an alkyl lithium compound such as n-butyl lithium at a reduced temperature, for example, between -100°C and -60°C in a solvent such as an ether (e.g. tetrahydrofuran). The mixture is then treated with a tri-substituted alkylborate compound such as triisopropylborate and the temperature conveniently brought up to room temperature. Subsequently, water may be added and the mixture treated with a strong mineral acid such as hydrochloric acid thus producing a compound of formula (VIa)

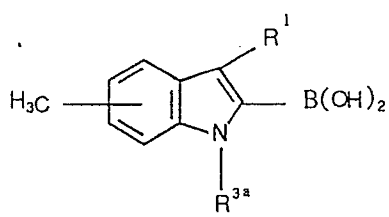

(VIa)

The intermediate compound of formula (VIa) is then reacted with a compound of formula (VII) in the presence of a palladium (O) compound such as tetrakis(triphenylphosphine)palladium (O) in a solvent such as an ether (e.g. dimethoxyethane), and in the presence of a base such as sodium carbonate. The reaction is conveniently effected at an elevated temperature, such as the reflux temperature of the solvent.

Indoles of formula (V) wherein $R^2$ is Ar and is attached at the 3-position on the indole ring may be prepared by cyclisation of a hydrazone of formula (VIII)

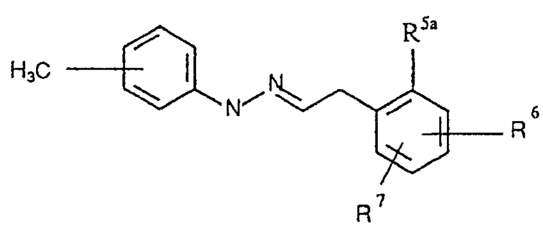

(VIII)

(wherein $R^{5a}$, $R^6$ and $R^7$ are as previously defined).

This Fischer synthesis of indoles can be effected using methods well known in the art. Thus, for example, the reaction may be effected in polyphosphoric acid or polyphosphate ester at a temperature between room temperature and 100°C.

Alternatively, indoles of formula (V) wherein $R^2$ is Ar and is attached at the 3-position on the indole ring may be prepared by a one-step reaction of a compound of formula (IX)

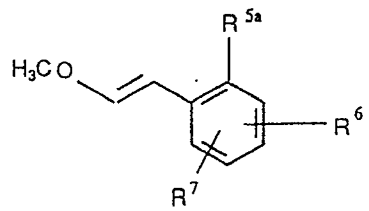

(IX)

with the appropriate tolyl hydrazine, preferably in the presence of an aqueous acid such as aqueous hydrochloric acid, in a solvent such as methanol and conveniently at room temperature.

Indoles of formula (V) wherein $R^{3a}$ is the group Ar (and consequently $R^2$ is a hydrogen or halogen atom) may be prepared by the reaction of a compound of formula (X)

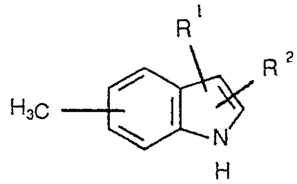

(X)

with a compound of formula (XI)

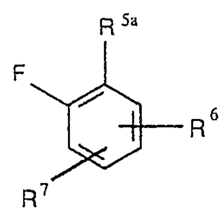

(XI)

in the presence of a base such as sodium hydride or potassium carbonate, in a solvent such as an ether e.g. tetrahydrofuran or a substituted amide e.g. dimethylformamide and conveniently, at a temperature between 0°C and the reflux temperature of the solvent. Indoles of formula (V) in which the substituent $R^5$ in the group Ar represents a C-linked tetrazolyl group may be prepared from a precursor of a compound of formula (V) wherein the substituent $R^5$ represents a nitrile group using the reagents and conditions described in process (C).

Similarly, intermediates of formula (VII) wherein $R^5$ represents a C-linked tetrazolyl group may be prepared from a compound of formula (XII)

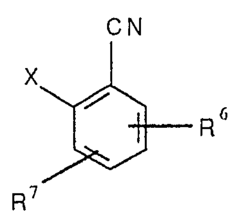

(XII)

(wherein X is as previously defined) followed where necessary by protection of any reactive groups, using methods well-known in the art such as those described in process (C).

Compounds of formula (V) in which the substituent $R^5$ in the group Ar is $-NHSO_2CF_3$ may be prepared from an precursor of a compound of formula (V) wherein the substituent $R^5$ is an amino group using the reagents and conditions described in process (D).

Similarly, intermediates of formula (VII) wherein $R^{5a}$ represents $-NHSO_2CF_3$ may be prepared from a

compound of formula (XIII).

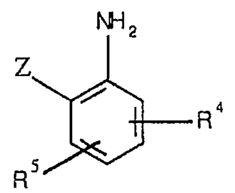

(XIII)

(followed, where necessary, by the protection of any reactive groups) using methods well known in the art such as those described in process (D).

It will be appreciated that indoles of formula (V) in which $R^1$ represents a hydrogen atom may be converted into compounds of formula (V) in which $R^1$ represents the group methyl (via hydrogenolysis of the Mannich base), -CHO (by reaction with N-disubstituted formamides and phosphorus oxychloride) or -$COR^4$ (wherein $R^4$ is as defined in general formula (I)) using techniques well known in the art, such as those described in "Properties and Reactions of Indoles" in Indoles by W.J. Houlihan (Ed), Vol, 1, p. 70, Wiley Interscience (1972), "Electrophilic Substitution Reactions on the Indole Ring" in The Chemistry of Indoles by R.I. Sundberg, Chapter 1, p.1 and p. 56, Academic Press (1970) or "Indoles : Reactions and Synthesis" in Heterocyclic Chemistry by J.A. Joule and G.F. Smith, Chapter 23, p. 257, Van Nostrand Reinhold Company, London (1972).

Intermediates of formula (VIII) may be prepared by the condensation of a compound of formula (XIV)

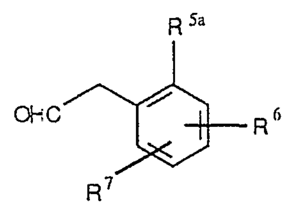

(XIV)

with the appropriate tolyl hydrazine using methods well known in the art, for example, in a solvent such as ethanol, and conveniently at room temperature.

The imidazoles of formula (III) may be prepared as described in European Specification No. 0253310A and in US Patent No. 4355040 or by methods analogous to those described therein. The content of these references is hereby incorporated by reference.

Intermediates of formulae (VI), (VII), (VIII), (IX), (X), (XI), (XII), (XIII) and (XIV) are either known compounds or may be prepared by methods analogous to those used for the preparation of the known compounds.

The following examples illustrate the invention. Temperatures are in $^{0}$C. "Dried" refers to drying using magnesium sulphate. Thin layer chromatography (t.l.c.) was carried out on silica and column chromatography was carried out on silica (Merck 9385), using one of the following solvent systems : A - dichloromethane : hexane, B -ether : hexane, C - dichloromethane : ethyl acetate, D - ether dichloromethane, E - dichloromethane : ethanol : conc. aqueous ammonia or F - ethyl acetate : hexane. The following abbreviations are used : THF - tetrahydrofuran; DME - dimethoxyethane; AIBN - azobisisobutyronitrile; DMF -dimethylformamide; DMAP - 4-dimethylaminopyridine; TFA - trifluoroacetic acid

Intermediate (1)
1,1-Dimethylethyl 5-methyl-1H-indole-1-carboxylate

Di-t-butyl dicarbonate (2.00ml) was added to a stirred solution of 5-methylindole (0.99g) and DMAP (100mg) in dry dichloromethane (40ml) at room temperature under nitrogen. After 1h the solvent was evaporated *in vacuo* and the residue purified by chromatography, eluting with System A (1:3) to give the *title compound* as a colourless oil.
t.l.c. System A (1:3) Rf 0.35.

Intermediate (2)
1,1-Dimethylethyl 2-borono-5-methyl-1H-indole-1-carboxylate

n-Butyl lithium (1.52M in hexane; 5.0ml) was added to a solution of Intermediate (1) (97mg) in dry THF (20ml) stirred at -78°C under nitrogen. After 90min, triisopropylborate (5.0ml) was added. After a further 2h the solution was allowed to warm to room temperature. After 60h at room temperature the mixture was quenched with water and acidified with iced sulphuric acid (ca 1M, 50ml). The mixture was then extracted with dichloromethane (2x20ml) and the combined extracts dried, filtered and evaporated in vacuo. The residue was purified by chromatography eluting with System B (1:1) to give a pale orange powder, which was washed with hexane to give the title compound as a white powder (298mg).
t.l.c. System B (1:2), Rf 0.3.

Intermediate (3)
1,1-Dimethylethyl 2-[2-(methoxycarbonyl)phenyl]-5-methyl-1H-indole-1-carboxylate

n-Butyl lithium (1.62M, in hexane; 25.0ml) was added to a stirred solution of Intermediate (1) (5.57g) in dry THF (100ml) at -78°C under nitrogen. After 90min triisopropylborate (25.0ml) was added, and stirring continued for 1h. The solution was then allowed to warm to room temperature before adding aqueous hydrochloric acid (2M, 250ml). The solution was extracted with ether (3x100ml) and the combined extracts dried, filtered and concentrated in vacuo. The residual solution (10ml) was cooled overnight and the resultant precipitate collected to give an off-white powder, Intermediate (2) (3.15g). This powder was added to a vigorously stirred mixture of methyl 2-bromobenzoate (2.50g) and tetrakis(triphenylphosphine) palladium (O) (250mg) in aqueous sodium carbonate (2M, 15ml) and dimethoxyethane (30ml), heated at reflux under nitrogen. After 90min the mixture was cooled to room temperature and ether (50ml) added. The organic phase was separated, washed with aqueous sodium hydroxide (2M, 50ml), dried, filtered and concentrated in vacuo. The residual oil was purified by chromatography eluting with System B (1:9) to give the title compound as a pale yellow oil (2.79g).
t.l.c. System B (1:9) Rf 0.3.

Intermediate (4)
1,1-Dimethylethyl 3-bromo-5-(bromomethyl)-2-[2-(methoxycarbonyl)- phenyl]-1H-indole-1-carboxylate

A mixture of Intermediate (3) (2. 195g), AIBN (250mg) and N-bromosuccinimide (2.20g) in carbon tetrachloride (40ml) was stood at room temperature for 1h in the dark, then heated at reflux under nitrogen whilst irradiating with a 150W sunlamp. The solvent was evaporated in vacuo after 3h and the residue purified by chromatography eluting with System B (1:4) to give a yellow solid. Recrystallisation from System B gave the title compound as pale yellow needles (1.86g) m.p. 140-142°C.
t.l.c. System B (1:9) Rf 0.25.

Intermediates (5) and (6)
1,1-Dimethylethyl 3-bromo-5-[[2-butyl-4-chloro-5-(hydroxymethyl)-1H-imidazol-1-yl]methyl]-2-[2-(methoxycarbonyl)phenyl]-1H-indole-1-carboxylate; and 1,1-Dimethylethyl 3-bromo-5-[[2-butyl-5-chloro-4-(hydroxymethyl)-1H- imidazol-1-yl]methyl]-2-[2-(methoxycarbonyl)phenyl]-1H-indole-1- carboxylate

Intermediate (4) (1.542g) was added to a stirred solution of the 2-butyl-5-chloro-1H-imidazole-4-methanol (550mg) and sodium methoxide (163mg) in dry DMF (20ml) at room temperature under nitrogen. After 60h the solvent was evaporated in vacuo and the residual oil purified by chromatography eluting with System C (4:1 → 1:1) to give a yellow oil, which on trituration with ether gave Intermediate (5) as a pale yellow solid (774mg), m.p. 160-165°C (dec.)
t.l.c. ether Rf 0.6.
and secondly Intermediate (6) as a yellow powder (268mg), m.p. 155 - 160°C (dec.)
t.l.c. ethyl acetate Rf 0.5.

Intermediate (7)
1,1-Dimethylethyl 3-bromo-5-[(2-butyl-1H-benzimidazol-1-yl)methyl]-2- [2-(methoxycarbonyl)phenyl]-1H-indole-1-carboxylate

A solution of 2-n-butylbenzimidazole (200mg), sodium methoxide (60mg) and Intermediate (4) (513mg)

in dry DMF (10ml) was stirred at room temperature under nitrogen for 60h. The solvent was removed in vacuo and the residue purified by chromatography eluting with System D (1:8) to give the title compound as a yellow foam (303mg).
t.l.c. System D (1:5), Rf 0.4.

Intermediate (8)
1,1-Dimethylethyl 5-[(2-butyl-1H-benzimidazol-1-yl)methyl]-2-[2-(methoxycarbonyl)phenyl]-1H-indole-1-carboxylate

A solution of Intermediate (7) (181mg) in 95% ethanol (20ml) was hydrogenated over palladium (50% aqueous paste, 10% on charcoal; 100mg) under ambient conditions for 3h. The catalyst was removed by filtration and the filtrate then concentrated in vacuo. Chromatography eluting with System E (200:8:1) gave the title compound as a colourless gum (147mg).
t.l.c. System D (1:2) Rf 0.65.

Intermediate (9)
1,1-Dimethylethyl 3-bromo-5-[(2-butyl-1H-imidazol-1-yl)methyl]-2-[2-(methoxycarbonyl)phenyl]-1H-indole-1-carboxylate

Intermediate (4) (500mg) was added to a stirred solution of 2-n-butylimidazole (150mg) and sodium methoxide (60mg) in dry DMF (10ml) at room temperature under nitrogen. After 60h the solvent was evaporated in vacuo and the residue purified by chromatography eluting with System D (1:1) to give a yellow oil. A solution of this oil in ether (10ml) was washed with aqueous sodium carbonate (2M, 10ml), dried, filtered and evaporated in vacuo to give the title compound as a white foam (432mg).
t.l.c. System D (1:1) Rf 0.3 detection.

Intermediate (10)
Methyl 2-(5-methyl-1H-indol-1-yl)benzoate

Sodium hydride (180mg), was added portionwise to a solution of 5-methyl indole (800mg), in DMF (10ml) under nitrogen. The suspension was stirred at room temperature for 45 minutes then methyl 2-fluorobenzoate (940mg) added and the resultant green mixture stirred at room temperature for 18h. A further portion of sodium hydride (60mg) was added and the suspension stirred at room temperature for 24h. Water (5ml) was added cautiously and the mixture was concentrated in vacuo. Ethyl acetate (5ml) was added and the organic phase was separated. The aqueous phase was extracted with ethyl acetate (2x5ml) and the combined organic phases dried and evaporated to give a dark purple oil (2.96g). Chromatography eluting with System B (1:4) gave a colourless oil (665mg). This was treated with ditert-butyldicarbonate (0.59g) and DMAP (29.3mg) in dichloromethane (40ml) to derivatise residual starting material. The solution was stirred at room temperature for 18h. The solvent was removed in vacuo and the residue was purified by chromatography eluting with System B (1:10) to give the title compound as a colourless oil (395mg).
t.l.c. System B (1:2), Rf 0.62.

Intermediate 11
Methyl 2-[2,3-dibromo-5-(bromomethyl)-1H-indol-1-yl]benzoate

A solution of Intermediate (10) (300mg), in carbon tetrachloride (20ml) was treated with N-bromosuccinimide (225mg) and stirred at room temperature in the dark for 30 minutes. The pale yellow solution was treated with a further portion of N-bromosuccinimide (225mg) and AIBN (21mg), irradiated with a 150W lamp and heated at reflux for 1h. The suspension was allowed to cool, the precipitate was filtered off and the solution was evaporated to give the title compound as a pink gum(570mg).
t.l.c. System F (1:2), Rf 0.60.

Intermediate (12)
Methyl 2-[2,3-dibromo-5-[[2-butyl-4-chloro-5-(hydroxymethyl)-1H-imidazol-1-yl]methyl]-1H-indol-1-yl]-benzoate

A solution of Intermediate (11) (ca. 165mg), in dry DMF (5ml) was added to a solution of 2-butyl-5-chloro-1H-imidazole-4-methanol (183mg), in DMF (15mg) previously treated with sodium methoxide (70mg),

and the solution was stirred at room temperature for 18h. The DMF was removed in vacuo, and the residue partitioned between water (10ml) and ether (8ml). The aqueous phase was extracted with ether (2 x 8ml) and the organic phases combined, washed with brine (10ml), dried and evaporated to give a yellow gum. Purification by chromatography eluting with System F (1:1) gave the title compound as a pale orange gum (140mg).
T.l.c. System F (1:2), Rf 0.38.

Intermediate (13)
1,1-Dimethylethyl 2-(2-cyanophenyl)-5-methyl-1H-indole-1-carboxylate

A stirred suspension of 2-bromobenzonitrile (2.6g) in DMF (80ml) and 8% sodium bicarbonate (70ml) was heated to 60° before Intermediate (2) (6.5g) and tetrakis(triphenylphosphine) palladium (0)(0.86g) were added. The mixture was stirred at reflux for 3h before being cooled and partitioned between ethyl acetate (100ml) and water (60ml). The organic phase was then dried and concentrated in vacuo to afford a red oil (8.9g). Chromatography eluting with System B (1:6) afforded the title compound as a pink solid (2.7g).
T.l.c. System B (1:2), Rf 0.7

Intermediate (14)
1,1-Dimethylethyl 5-[(2-butyl-4-chloro-5-hydroxymethyl-1H-imidazole-1-yl)methyl]-2-(2-cyanophenyl)-1H-indole-1-carboxylate

N-Bromosuccinimide (0.238g) was added to a stirred solution of Intermediate (13) (0.435g) in carbon tetrachloride (25ml). AIBN (61mg) was added and the mixture heated at reflux for 40min whilst being irradiated with a 200 Watt lamp. Further N-bromosuccinimide (63mg) was added and heating continued for a further 10min. The cooled suspension was-filtered and concentrated in vacuo to afford a yellow oil (1.05g). A mixture of 2-butyl-4-chloro-5-hydroxymethylimidazole (0.286g) and the yellow oil (1.05g) in DMF (17ml) containing sodium methoxide (0.115g) was stirred overnight at room temperature. This was concentrated in vacuo to afford an orange solution (10ml) which was partitioned between ethyl acetate (100ml) and water (50ml). The organic phase was washed with water (3x50ml), dried and concentrated in vacuo to afford an orange oil (1.12g). Chromatography, eluting with System E (200:8:1) gave the title compound as a yellow oil (0.173g).
T.l.c. System E (150:8:1), Rf 0.3

Intermediate (15)
2-[5-[[2-Butyl-4-chloro-5-hydroxymethyl-1H-imidazol-1-yl]methyl]-1H- indol-2-yl]benzonitrile

TFA (1 ml) was added dropwise to a stirred solution of Intermediate (14) (0.45g) in dichloromethane (15ml) at 0°C. After warming to room temperature, further TFA (1ml) was added and stirring continued for 1h. The red solution was concentrated in vacuo, chloroform (35ml) was added and the solution washed with 8% sodium bicarbonate (50ml). The separated chloroform phase was dried and concentrated in vacuo to afford a red oil (0.6g) which was redissolved in dichloromethane (25ml), TFA (2ml) added and stirring continued for 2h. The solution was concentrated in vacuo, the residue taken up in chloroform (35ml), washed with 8% sodium bicarbonate (2x35ml), dried and concentrated in vacuo to afford a red oil (0.67g). Chromatography eluting with System E (200:8:1) gave the title compound (0.1 63g) as a pale yellow foam.
T.l.c. System E (150:8:1), Rf 0.4

Intermediate (16)
1,1-Dimethylethyl 5-methyl-2-(2-nitrophenyl)-1H-indole-1-carboxylate

A mixture of Intermediate (2) (0.4g), 2-bromonitrobenzene (0.3g), and tetrakis(triphenylphosphine) palladium (0) (75mg) in DME (20ml) and sodium carbonate (2N, 10ml) was heated at reflux for 2 days. Ether (30ml) was added to the cooled solution and the organic layer separated. The aqueous phase was further extracted with ether (2x30ml), the combined organic extracts dried and the solvent removed in vacuo. The residue was purified by chromatography eluting with System B (1:15) to give the title compound as a yellow gum (0.27g).
T.l.c. System B (1:15) Rf 0.25

Intermediate (17)

1,1-Dimethylethyl 5-(bromomethyl)-2-(2-nitrophenyl)-1H-indole -1-carboxylate

A mixture of Intermediate (16) (0.8g), N-bromosuccinimide (0.48g) and AIBN (100mg) in carbon tetrachloride (30ml) was heated at reflux for 4h under a 200W lamp. The cooled reaction was filtered, the solvent evaporated in vacuo, and the residue purified by chromatography, eluting with System B (1:10) to give the title compound as a yellow gum (0.5g).
T.l.c. System B (1:15) RF 0.2.

Intermediate (18)
1,1-Dimethylethyl 5-[(2-butyl-1H-imidazol-1-yl)methyl]-2-(2-nitrophenyl)-1H-indole-1-carboxylate

Intermediate (17) (0.45g) was added to a solution of 2-butyl imidazole (0.15g) and sodium methoxide (60mg) in DMF (20ml), and the resulting mixture stirred for 48h. Solvent was removed in vacuo, and the residue partitioned between water (20ml) and dichloromethane (20ml). The organic layer was separated and the aqueous layer further extracted with dichloromethane (2x20ml). Organic extracts were dried, solvent removed in vacuo, and the residue purified by chromatography eluting with System E (300:8:1) to give the title compound as a yellow oil (0.3g).
T.l.c. System E(300:8:1) Rf 0.29

Intermediate (19)
5-[(2-Butyl-1H-imidazol-1-yl)methyl]-2-(2-nitrophenyl)-1H-indole

A solution of Intermediate (18) (0.5g) in dichloromethane (5ml) and TFA (1ml) was stirred at room temperature for 1h. Solvent was removed in vacuo and the residue purified by chromatography eluting with System E (200:8:1) to give the title compound as a yellow gum (330mg).
T.l.c. System E (200:8:1) Rf 0.2

Intermediate (20)
2-[5-[(2-Butyl-1H-imidazol-1-yl)methyl]-1H-indol-2-yl]-benzenamine

A solution of Intermediate (19) (0.33g) in 95% ethanol (50ml) was hydrogenated over palladium (10% on charcoal, 50% aqueous paste, 0.2g) for 8h. Catalyst was removed by filtration and the solvent removed in vacuo to give the title compound as a colourless oil (0.3g).
T.l.c. System E (100:8:1) Rf 0.5.

Intermediate (21)
1,1-Dimethylethyl 3-bromo-2-[2-(methoxycarbonyl)phenyl]-5-methyl-1H- indole-1-carboxylate

A mixture of Intermediate (3) (2.02g) and N-bromosuccinimide (1.05g) in carbon tetrachloride (40ml) was heated at reflux under nitrogen for 0.5h. The mixture was cooled to room temperature, filtered and concentrated in vacuo. Chromatography of the residual oil, eluting with System B (1:9) gave a pale yellow oil, which was crystallised from hexane to give the title compound as colourless needles (1.61g).
m.p. 100-102°C

Intermediate (22)
Methyl 2-[3,6-dibromo-5-[(2-butyl-1H-imidazol-1-yl)methyl]-1-methyl- 1H-indol-2-yl]benzoate

A solution of Intermediate (21) (1.60g) in TFA (3ml) and dichloromethane (3ml) was stood under nitrogen at room temperature for 1h. The solution was concentrated in vacuo and ether (10ml) added. The solution was then washed with aqueous sodium carbonate (2M, 2x10ml), dried, filtered and concentrated in vacuo to give a yellow oil. Sodium hydride (60% dispersion in oil; 200mg) was added to a stirred solution of this oil in methyl iodide (0.30ml) and THF (20ml) at room temperature under nitrogen. After 1h methanol (1ml) was added before concentrating in vacuo. Chromatography, eluting initially with chloroform:hexane and then with chloroform gave a yellow oil. A mixture of this oil and N-bromosuccinimide (0.50g) in carbon tetrachloride (40ml) was heated at reflux for 30min, before the mixture was cooled to room temperature, filtered and concentrated in vacuo. Chromatography of the residue, eluting with chloroform/hexane (2:3) gave a yellow oil which was further purified by chromatography eluting with System B(1:5) to give a yellow oil. A solution of this oil, N-bromosuccinimide (200mg) and AIBN (50mg) in carbon tetrachloride (20ml) was

heated at reflux under nitrogen whilst irradiating with a 150W lamp for 0.5h. The mixture was cooled to room temperature, filtered and then concentrated in vacuo to give a yellow solid. A solution of this solid was dissolved in dichloromethane (1 ml) and ether (2ml) added. The mother liquor was removed and concentrated in vacuo. Ether (1 ml) was added followed by hexane (5ml) and the precipitate collected to give a yellow solid (231mg). Sodium methoxide (33mg) was added to a solution of this solid and 2-butylimidazole (100mg) in dry DMF (10ml), stirred at room temperature. under nitrogen. After 60h the solvent was concentrated in vacuo, the residue taken up in dichloromethane (10ml), washed with water (5ml), dried, filtered and concentrated in vacuo to give a brown gum. Chromatography, eluting with System E (300:8:1) gave the title compound as a yellow gum (129mg).
T.l.c. System E (300:8:1) Rf 0.3

Intermediate (23)
Methyl 2-[5-(2-butyl-1H-imidazol-1-yl)-1-methyl-1H-indol-2-yl]benzoate

A solution of Intermediate (22) (243mg) in 95% ethanol (40ml) was hydrogenated over palladium (50% wet paste; 10% on charcoal, 200mg) under ambient conditions for 3h. The catalyst was removed by filtration through celite and the solvent evaporated in vacuo. Dichloromethane (20ml) was added and the mixture washed with aqueous sodium hydroxide (2M, 20ml), dried, filtered and evaporated in vacuo to give the title compound as a yellow oil (155mg).
T.l.c. System D (1:1) RF 0.3

Intermediate (24)
1,1-Dimethylethyl 5-[(2-butyl-1H-imidazol-1-yl)methyl]-2-[2-(methoxy carbonyl)phenyl]-1H-indole-1-carboxylate

A solution of Intermediate (9)(1.4g) in 95% ethanol (50ml) was hydrogenated over a palladium catalyst (10% on charcoal; 50% aqueous paste, 0.5g) under ambient conditions for 4h. The catalyst was removed by filtration and the solution concentrated in vacuo. The residue was purified by chromatography eluting with System E (300:8:1) to give the title compound as a slightly coloured oil (1.0g).
T.l.c. System E (300:8:1) Rf 0.3.

Intermediate (25)
Methyl 2-[5-[(2-butyl-1H-imidazol-1-yl)methyl]-1H-indol-2-yl]benzoate

A solution of Intermediate (24) (600mg) in dichloromethane (1 0ml) and TFA (2ml) was stirred at ambient temperature overnight. Solvent was removed in vacuo and the residue purified by chromatography eluting with System E (300:8:1) to give the title compound as a yellow foam (400mg).
T.l.c. System E (300:8:1) Rf 0.27

Intermediate (26)
Methyl 2-[5-[(2-butyl-1H-imidazol-1-yl)methyl-3-formyl]-1H-indol-2-yl]benzoate

Phosphorus oxychloride (0.1ml) was added dropwise to ice-cold DMF (2ml). The resulting solution added to an ice-cold solution of Intermediate (25) (0.37g) in DMF (5ml), and the mixture stirred for 4h. Solvent was partially removed in vacuo and the residue partitioned between sodium bicarbonate solution (2N, 10ml) and dichloromethane. The organic layer was separated, the aqueous layer further extracted with dichloromethane (5x15ml) and the dried organic extracts evaporated in vacuo. The residue was purified by chromatography, eluting with System E (300:8:1) to give the title compound as a yellow solid (330mg) m.p. 153-154°C.

Intermediate (27)
Methyl 2-[3-bromo-5-[(2-butyl-1H-imidazol-1-yl)methyl]-1H-indol-2-yl]benzoate

A solution of Intermediate (9) (567mg) in TFA (1ml) and dichloromethane (5ml) was stood at room temperature for 78h. The solution was washed with aqueous sodium hydroxide (2N; 25ml), dried, filtered and concentrated in vacuo to give the title compound as a red oil (472mg).
T.l.c. System E (300:8:1) Rf 0.3

Intermediate (28)
Methyl 2-[3-bromo-5-[(2-butyl-1H-imidazol-1-yl)methyl]-1-methyl-1H-indol-2-yl]benzoate

Sodium hydride (60% in oil; 50mg) was added to a solution of Intermediate (27) (460mg) in dry THF (10ml) and stood at room temperature under nitrogen. After 1h, methyl iodide (70μl) was added dropwise over 5min. After a further 16h, the solvent was removed in vacuo and the residue purified by chromatography, eluting with dichloromethane:ethanol (10:0→10:1) to give the title compound as a yellow oil (299mg).
T.l.c. ether Rf 0.1

Intermediate (29)
Methyl 2-(5-methyl-1H-indol-3-yl)benzoate

A solution of methyl 2-(2-methoxyethenyl)benzoate (4.05g) in methanol (70ml) and 2N hydrochloric acid (14ml) was treated with p-tolylhydrazine hydrochloride (3.38g) and the dark green solution was stirred at room temperature for 36h. Water (70ml) and brine (30ml) were added and the mixture extracted with ethyl acetate (4x55ml). The dried organic phase was evaporated to give the title compound as a dark green-blue solid (5.83g).
T.l.c. System B (1:2) Rf 0.33

Intermediate (30)
1,1-Dimethylethyl 3-[2-(methoxycarbonyl)phenyl]-5-methyl-1H-indole-1-carboxylate

A solution of Intermediate (29)(5.83g) in dichloromethane (30ml) was treated with di-tert-butyl dicarbonate (5.06g) and DMAP (795mg) and the resultant dark red solution was stirred at room temperature for 3h. The solvent was removed in vacuo and the dark red residue was purified by chromatography System B (1:2) to give the title compound as a red oil (2.51 g).
T.l.c. System B (1:1) Rf 0.65

Intermediate (31)
1,1-Dimethylethyl 5-(bromomethyl)-3-[2-(methoxycarbonyl)phenyl]-1H- indole-1-carboxylate

A solution of Intermediate (30)(1.95g) in carbon tetrachloride (30ml) was treated with N-bromosuccinimide (950mg) and AIBN (395mg), heated at reflux and irradiated with a 200W lamp. The mixture was allowed to cool to room temperature and was then filtered and evaporated. The residue was purified by chromatography eluting with System B (1:10) to give the title compound as an orange gum (1.93g).
n.m.r δ($CDCl_3$) 1.68 (9H,s), 3.6 (3H,s), 4.58 (2H,s), 7.32-7.7 (6H,m), 7.95 (1H,m), 8.16(1H,br).

Intermediate (32)
1,1-Dimethylethyl 5-[2-butyl-4-chloro-5-(hydroxymethyl)-1H-imidazol -1-yl]methyl-3-[2-(methoxycarbonyl)-phenyl]-1H-indole-1-carboxylate

Intermediate (31) (1.9g) was added to a stirred solution of 2-butyl-5-chloro-1H-imidazole-4-methanol (820mg) and sodium methoxide (235mg) in DMF (30ml). The solution was stirred at room temperature. for 18h under nitrogen. The solvent was removed in vacuo and the residue was partitioned between water (20ml) and diethyl ether (2x20ml). The dried organic phase was evaporated to give an orange gum (2.4g). Purification by chromatography eluting with System E (400:8:1) gave the title compound as an orange gum (760mg).
T.l.c. System E (300:8:1) Rf 0.36.

Intermediate (33)
1,1-Dimethylethyl 4-methyl-1H-indole-1-carboxylate

A solution of 4-methylindole (1.52g) and di-tert-butyl dicarbonate (2.92ml) in dichloromethane (25ml) was cooled to 0°C under nitrogen and treated with DMAP (0.2g). The resulting solution was warmed to room temperature and stirring continued overnight. Solvent was evaporated and the residue dissolved in hexane (50ml), washed with 2N HCl (6x20ml) and dried. Filtration and evaporation gave the title compound as an orange oil, (2.698g).
T.l.c. System B (1:9) Rf 0.8

Intermediate (34)
1,1-Dimethylethyl 2-borono-4-methyl-1H-indole-1-carboxylate

A solution of Intermediate (33), (2.56g) in THF (50ml) was cooled to -78°C with stirring under nitrogen and treated dropwise with n-butyl lithium (9.2ml) maintaining the temperature below -70°C. The solution so formed was stirred at -78°C for a further hour before treating with triisopropylborate (7.70ml) maintaining the temperature below -70°C. After an hour the solution was warmed gradually to room temperature before quenching with 2N HCl (50ml). The solution was extracted with ether (3x30ml) and the combined organic extracts dried and evaporated. Hexane (50ml) was added, the solution evaporated and the residue triturated with hexane (50ml). The precipitate was collected by filtration to give the title compound as a greyish powdered solid (1.17g).
T.l.c. System B (1:9) Rf 0.2.

Intermediate (35)
1,1-Dimethylethyl 2-[2-(methoxycarbonyl)phenyl]-4-methyl-1H-indole -1-carboxylate

A solution of methyl 2-bromobenzoate (1.17g) in DME (20ml) and 2N sodium carbonate (6ml) was heated to reflux with vigorous stirring under nitrogen. Tetrakis(triphenylphosphine) palladium (0) (100mg) and Intermediate (34) (1.43g) were added and reflux under nitrogen continued. After 4h the reaction was cooled to room temperature and diluted with ether (50ml). The organic extract was separated and washed with water (3x50ml) dried, and evaporated to give a dark orange oil (2.71 g). This was purified by column chromatography eluting with System B (1:9) to give the title compound as a yellow oil (0.581g).
T.l.c. System B (1:9) Rf ~0.45

Intermediate (36)
1,1-Dimethylethyl 3-bromo-4-(bromomethyl)-2-[2-(methoxycarbonyl) phenyl]-1H-indole-1-carboxylate

A solution of Intermediate (35) (0.22g) in carbon tetrachloride (5ml) was treated with N-bromosuccinimide (0.108g) and heated to reflux. After 30min, a further equivalent of N-bromosuccinimide was added, the solution treated with AIBN (~5mg) and heated to reflux whilst irradiating with a 200W tungsten bulb. Reaction was complete after a further 30min. Evaporation of solvent gave a yellow solid (0.48g) which was purified by column chromatography eluting with System B (1:9) to give the title compound as a yellow solid (0.246g).
T.l.c. System B (1:9) Rf 0.4

Intermediate (37)
1,1-Dimethylethyl 3-bromo-4-[[2-butyl-1H-imidazol-1-yl]methyl]-2-[2-(methoxycarbonyl)phenyl]-1H-indole-1-carboxylate

A solution of Intermediate (36) (0.074g) in DMF (3ml) was treated with sodium methoxide (0.024g). Stirring at room temperature was continued for 30min before addition of a solution of 2-butyl imidazole (0.23g) in DMF (3ml). Stirring at room temperature was continued overnight. The solvent volume was reduced and the residue dissolved in ethyl acetate (30ml) and washed with water (2x30ml) before drying. Filtration and evaporation gave a yellow oil (0.39g) which was purified by column chromatography eluting with System E (300:8:1) to give the title compound as a yellow oil (0.179g).
T.l.c. System D (1:1) Rf 0.2.

Intermediate (38)
1,1-Dimethylethyl 3-bromo-5-[[2-butyl-4-chloro-5-(ethoxycarbonyl)-1H-imidazol-1-yl]methyl]-2-[2-(methoxycarbonyl)phenyl]-1H-indole-1-carboxylate

A solution of ethyl 2-butyl-4-chloro-1H-imidazole-5-carboxylate (0.10g) and Intermediate (4) (0.34g) in DMF (7ml) was treated with sodium hydride (0.0146g) and stirred at room temperature overnight. The solvent was removed and the residue partitioned between dichloromethane and water. The organic layer was washed with water, dried, filtered and evaporated to give a yellow gum. Purification by column chromatography eluting with System B (1:3) afforded the title compound as a white solid (0.1g).
T.l.c System B (1:2), Rf 0.25.

Intermediate (39)
2-(2-Cyanophenyl)-5-methyl-1H-indole

Trifluoroacetic acid (1.4ml) was added to a stirred solution of Intermediate (13) (5.9g) in dichloromethane (150ml) at 0° under nitrogen. After 30min, further trifluoroacetic acid (1.4ml) was added and stirring continued overnight. Further trifluoroacetic acid (3.5ml) was added and stirring continued for 60 hours. The dark red solution was concentrated in vacuo to afford a residue which was dissolved in chloroform (150ml) and washed with 8% sodium bicarbonate (2x70ml). The chloroform extract was dried and concentrated in vacuo to afford a dark red oil (6.1g). Purification by chromatography eluting with System B (1:5) → (1:2) afforded the title compound (1.65g) as a yellow solid.
T.l.c., System, B (1:1), Rf 0.3

Intermediate (40)
5-Methyl-2-[2-(1H-tetrazol-5-yl)phenyl]-1H-indole

A mixture of Intermediate (39) (0.96g) and tri-n-butyl tin azide (5.3g) was stirred at 165° for 1h. After cooling, the dark solution was diluted with methanol (30ml) before 2N sodium hydroxide (30ml) and water (30ml) were added. The cloudy green solution was washed with ether (3x40ml) before being acidified with 5N hydrochloric acid (50ml) and extracted with ethyl acetate (3x50ml). The combined organic extracts were dried and concentrated in vacuo to afford a green foam (0.95g) which was azeotroped with toluene (2x30ml) to afford the title compound (0.935g) as a green foam.
T.l.c., System B (1:1), Rf 0.2 (Streak)

Intermediate (41)
3-Bromo-5-methyl-2-[2-(1H-tetrazol-5-yl)phenyl]-1H-indole

Bromine (1M solution in carbon tetrachloride, 2ml) was added dropwise to a cooled solution of Intermediate (40) (0.93g) at 8° under nitrogen. After stirring for 2h at 20° and standing overnight, further bromine (0.5ml) was added. After 1h, the dark green solution was diluted with chloroform (40ml) and concentrated in vacuo to afford the title compound (1.32g) as a green oil. T.l.c., ether, Rf 0.1 (Streak)

Intermediate (42)
3-Bromo-5-methyl-2-[2-[2-(triphenylmethyl)-2H-tetrazol-5-yl]phenyl]-1H-indole

A mixture of triphenylmethyl chloride (0.7g) and DMAP (0.05g) was added to a solution of Intermediate (41) (1.3g) in dichloromethane (60ml) containing triethylamine (2ml) whilst being stirred at room temperature. The solution was then stirred for 6h before standing for 72h. The brown solution was concentrated in vacuo to afford a brown semi-solid residue (2.18g). Column chromatography eluting with System B (1:9) → (1:3) afforded the title compound (1.02g) as an off-white solid, m.p. 190°-192° (decomp.).
Assay Found: C,69.8; H,4.4; N,11.35;
$C_{35}H_{26}BrN_5.0.25H_2O$ requires C,70.0; H,4.4; N,11.65%

Intermediate (43)
1,1-Dimethylethyl 3-bromo-5-methyl-2-[2-[2-(triphenylmethyl)-2H-tetrazol-5-yl]phenyl]-1H-indole-1-carboxylate

Di-tert-butyl dicarbonate (0.38g) and Intermediate (42) (1.02g) were dissolved in dichloromethane (60ml) before DMAP (0.06g) was added. After stirring for 24h, the solution was concentrated in vacuo to afford a cream solid (1.47g). Column chromatography eluting with System B (1:4) afforded the title compound - (1.04g) as a white solid.
T.l.c., System B (1:1), Rf 0.75

Intermediate (44)
1,1-Dimethylethyl 3-bromo-5-bromomethyl-2-[2-[2-(triphenylmethyl)-2H-tetrazol 5-yl]phenyl]-1H-indole-1-carboxylate

N-Bromosuccinimide (0.282g) and dibenzoyl peroxide (0.06g) were added to a stirred solution of Intermediate (43) (1.04g) in carbon tetrachloride (75ml) at 70°. The mixture was then heated at reflux for 3h

whilst being irradiated with a 250 watt lamp. After cooling, the suspension was filtered and the filtrate concentrated in vacuo to afford the title compound (1.45g) as a white solid.
T.l.c., System B (1:1), Rf 0.7

Intermediate (45)
1,1-Dimethylethyl 3-bromo-5-[[2-butyl-4-chloro-5-(ethoxycarbonyl)-1H-imidazol-1-yl]methyl]-2-[2-[2-(triphenylmethyl)-2H-tetrazol-5-yl)phenyl]-1H-indole-1-carboxylate

Sodium hydride (60% dispersion in oil, 0.056g) was added to a stirred solution of ethyl 2-butyl-4-chloro-1H-imidazole-5-carboxylate (0.25g) in DMF (15ml) under nitrogen. After stirring for 45min, the solution was cooled to 0° before a solution of Intermediate (44) (1.45g) in DMF (25ml) was added dropwise over 15min. The solution was then stirred at room temperature for 60 hours. The pale yellow solution was partitioned between ethyl acetate (100ml), brine (50ml) and water (50ml). The separated aqueous phase was further extracted with ethyl acetate (1x60ml) and the combined organic extracts were washed with water (3x100ml), dried and concentrated in vacuo to afford an orange gum (1.45g). Column chromatography eluting with System B (1:3) afforded the title compound (0.52g) as a white foam.
T.l.c., System B (1:1), Rf 0.35

Intermediate (46)
1,1-Dimethylethyl 3-bromo-5-[[2-butyl-4-chloro-5-(ethoxycarbonyl)-1H-imidazol-1-yl]methyl]-2-[2-(1H-tetrazol-5-yl)phenyl]-1H-indole-1-carboxylate

Concentrated hydrochloric acid (1ml) was added dropwise to a stirred suspension of Intermediate (45) (0.085g) in methanol (10ml). After stirring overnight the solution was basified with 2N sodium hydroxide (8ml), water (10ml) added and the mixture was then extracted with ethyl acetate (3x15ml). The combined organic extracts were dried and concentrated in vacuo to afford a colourless oil (0.07g). Column chromatography eluting with ether:acetic acid (95:5) afforded the title compound (0.043g) as a white foam.
n.m.r.($CDCl_3$) 8.22δ (2H,m), 7.65δ (2H,m), 7.43δ (1H,m), 7. 15δ (1H,brs), 7.08δ (1H,dd), 5.65δ (2H,s), 4.27δ - (2H,q), 2.6δ (2H,t), 1.62δ (2H,m), 1.3δ (5H,m), 1.15δ (9H,s), 0.83δ (3H,t).

Intermediate (47)
1,1-Dimethylethyl 3-bromo-5-methyl-2-(2-nitrophenyl)-1H-indole-1-carboxylate

A mixture of Intermediate (16) (3.2g) and anhydrous sodium acetate (0.9g) in carbon tetrachloride (50ml) at 0°C was treated with a solution of bromine in carbon tetrachloride (1M; 10ml) dropwise over 5 minutes. Further bromine (1M; 2ml) was added and the mixture stirred at 0° for 4h. The reaction mixture was diluted with dichloromethane (50ml), washed with aqueous sodium thiosulphate solution (100ml; 10%), dried and evaporated in vacuo. The residue was crystallized from ethanol to give the title compound as a yellow solid (3.1g), m.p. 148.5-149.5°.

Intermediate (48)
1,1-Dimethylethyl 3-bromo-5-(bromomethyl)-2-(2-nitrophenyl)-1H-indole-1-carboxylate

A mixture of Intermediate (47) (0.31 5g) and N-bromosuccinimide (0.14g) in dry carbon tetrachloride (5ml) was heated under reflux, whilst being irradiated with a 200W light for 5h. The reaction mixture was filtered and the filtrate was washed with water (20ml), dried and evaporated to give the title compound as a yellow foam (0.4g).

Intermediate (49)
1,1-Dimethylethyl 3-bromo-5-[[2-butyl-4-chloro-5-(ethoxycarbonyl)-1H-imidazol-1-yl]methyl]-2-(2-nitrophenyl)-1H-indole-1-carboxylate

A solution of ethyl 2-butyl-4-chloro-1H-imidazole-5-carboxylate (1.15g) in dry DMF (30ml) was treated with sodium hydride (0.13g) and the mixture was stirred at ambient temperature for 30min. A solution of Intermediate (48) (0.4g) in dry DMF (10ml) was added at 0°C and the mixture was stirred at 0°C to ambient temperature for 20h. The solvent was evaporated in vacuo, and the residue was dissolved in ether (100ml) and washed with water (100ml), aqueous sodium bicarbonate solution (100ml; 8%), and aqueous lithium chloride solution (10%; 50ml). The organic solution was dried, filtered and evaporated in vacuo to

give an oily residue. The residue was purified by FCC eluting with System 15 (1:50) to give the title compound as a yellow foam (1.54g) 72-73°.

Intermediate (50)
Ethyl 1-[[3-bromo-2-(2-nitrophenyl)-1H-indol-5-yl]methyl]-2-butyl-4-chloro-1H-imidazole-5-carboxylate

A solution of Intermediate (49) (0.75g) in a mixture of dry dichloromethane (20ml) and TFA (4.5ml) at ambient temperature was stirred for 2h. The solvent was evaporated in vacuo and the residue was purified by FCC eluting with dichloromethane/methanol/ ammonia (300:8:1) to give the title compound as an orange foam (0.63g), m.p. 90.5-91.5°C.

Intermediate (51)
Ethyl 1-[[2-(aminophenyl)-3-bromo-1H-indol-5-yl]methyl]-2-butyl-4-chloro-1H-imidazole-5-carboxylate

A solution of Intermediate (50) (0.47g) in a mixture of conc. hydrochloric acid (6ml), water (6ml) and THF (20ml) was hydrogenated over 5% palladium on carbon (0.2g) at ambient temperature and pressure. The mixture was filtered and the filtrate was evaporated in vacuo. The aqueous residue was basified with aq. sodium carbonate (8%; 100ml) and the product was extracted with dichloromethane (2x75ml). The extract was dried and evaporated in vacuo. The residue was purified by FCC eluting with System B (1:1) to give the title compound as an off-white foam (0.3g), m.p. 77-80°C.

Example (1)
2-[3-Bromo-5-[[2-butyl-4-chloro-5-(hydroxymethyl)-1H-imidazol-1-yl]-methyl]-1H-indol-2-yl]benzoic acid

A solution of Intermediate (5) (202mg) and aqueous sodium hydroxide (2M, 1ml) in methanol (2ml) and THF (2ml) was stirred at room temperature under nitrogen for 20h. The solution was concentrated in vacuo before adding water (4ml) and hydrochloric acid (2M, 1ml). The precipitate was collected and dried in vacuo to give the title compound as a white powder (139mg) m.p. 150-180°C (dec.).
Assay Found: C,54.2; H,4.5; N,7.7.
$C_{24}H_{23}BrClN_3O_3.0.54H_2O$ requires C,54.7; H,4.6; N,8.0%.
Water Assay showed 1.85% $H_2O$w/w (≡ 0.54mol $H_2O$)

Example (2)
2-[3-Bromo-5-[[2-butyl-5-chloro4-(hydroxymethyl)-1H-imidazol-1-yl]- methyl]-1H-indol-2-yl]benzoic acid, 2,2,2-trifluoroacetate salt.

A solution of Intermediate (6) (242mg) and aqueous sodium hydroxide (2M, 1ml) in methanol (2ml) and THF (2ml) was stood at room temperature for 20h. The solution was concentrated in vacuo, hydrochloric acid (2M, 1ml) was added and the mixture extracted with ethyl acetate (3x5ml). The combined extracts were dried, filtered and evaporated in vacuo to give a yellow gum. TFA (1ml) and dry dichloromethane (5ml) were added and the solution stood at room temperature under nitrogen for 1h before concentrating in vacuo to give the title compound as a brown foam (160mg).
Assay Found: C,49.1; H,4.0; N,6.3.
$C_{24}H_{23}BrClN_3O_3.CF_3CO_2H$ requires C,49.5; H,3.8; N,6.7%.
n.m.r δ(DMSO-$d_6$) 0.87 (3H,t), 1.35 (2H,m), 1.57 (2H,m), 2.98 (2H,t), 4.48 (2H,s), 5.52 (2H,s), 7.04 (1H,dd), 7.3 (1H,brs), 7.43 (1H,d), 7.54 (1H,dd), 7.62 (1H,ddd), 7.72 (1H,ddd), 8.0 (1H,dd), 11.9 (1H,s), 12.8 (1H, vbrs).

Example (3)
2-[5-[(2-Butyl-1H-benzimidazol-1-yl)methyl]-1H-indol-2-yl]benzoic acid, monohydrochloride

A solution of Intermediate (8) (141mg) and aqueous sodium hydroxide (2M, 1ml) in methanol (3ml) was stood for 120h at room temperature. The solution was concentrated in vacuo and hydrochloric acid (2M, 2ml) added. The precipitate was collected and dried in vacuo to give the title compound as an off-white powder (87mg), m.p. 130-140°C (dec.)
. Assay Found: C,70.2; H,5.5; N,9.0.
$C_{27}H_{25}N_3O_2.HCl$ requires C,70.5; H,5.7; N,9.1%.

Example (4)

2-[3-Bromo-5-[(2-butyl-1H-benzimidazol-1-yl)methyl]-1H-indol-2-yl]- benzoic acid, monohydrochloride

A solution of Intermediate (7) (103mg) and aqueous sodium hydroxide (2M, 1ml) in methanol (4ml) was stirred at room temperature under nitrogen for 20h. The solvent was concentrated in vacuo and water (3ml) added. The solution was then acidified with hydrochloric acid (2M, 2ml) and the precipitate collected to give the title compound as a white powder (79mg) m.p. 160-170°C (dec.).

Assay Found: C,60.0; H,4.5; N,7.4.

$C_{27}H_{24}BrN_3O_2.HCl$ requires C,60.2; H,4.7; N,7.8%.

Example(5)

2-[5-[(2-Butyl-1H-imidazol-1-yl)methyl]-1H-indol-2-yl]benzoic acid

A solution of Intermediate (9) (300mg) in 95% ethanol (40ml) was hydrogenated over a palladium catalyst (10% on charcoal; 50% aqueous paste; 177mg) under ambient conditions for 4h. The catalyst was removed by filtration and the solution concentrated in vacuo. Dichloromethane (10ml) was added and the solution washed with aqueous sodium hydroxide (2M, 10ml), dried, filtered and evaporated in vacuo to give a white foam (265mg). A portion of this foam (242mg) was added to a mixture of methanol (6ml) and aqueous sodium hydroxide (2M, 2ml) and the solution heated at 60°C under nitrogen for 5h. The solution was concentrated in vacuo and hydrochloric acid (2M, 2ml) added. The precipitate was collected and dried in vacuo to give the title compound as a white powder (120mg) m.p. 110-120°C (dec.).

Assay Found: C 72.4; H,6.3; N,10.8.

$C_{23}H_{23}N_3O_2.0.2H_2O$ requires C,73.3; H,6.3; N,11.1%.

Water assay shows 0.89% $H_2O$w/w (≡ 0.2mol $H_2O$)

Example (6)

2-[3-Bromo-5-[(2-butyl-1H-imidazol-1-yl)methyl]-1H-indol-2-yl]benzoicacid

A solution of Intermediate (9) (125mg) in methanol (8ml) and aqueous sodium hydroxide (2M, 2ml) was stood at room temperature under nitrogen for 16h and at 60°C for 2h. It was then concentrated in vacuo. Hydrochloric acid (2M, 2ml) was added. The precipitate was then collected and dried in vacuo to give the title compound as a white powder (91mg) m.p. 140-150°C (dec.).

Assay Found: C,59.5; H,4.9; N,8.9.

$C_{23}H_{22}BrN_3O_2.0.6H_2O$ requires C,59.6; H,5.1; N,9.0%.

Water Assay shows 2.46% $H_2O$w/w (≡ 0.6mol $H_2O$)

Example (7)

2-[2,3-Dibromo-5-[[2-butyl-4-chloro-5-(hydroxymethyl)-1H-imidazol-1-yl]methyl]-1H-indol-1-yl]benzoic acid, 2,2,2-trifluoroacetate (1:1) salt

A solution of Intermediate (12) (130mg) in methanol (5ml) was treated with 2N sodium hydroxide solution (2ml) and stirred at room temperature for 36h. The methanol was removed in vacuo, the residue was diluted with water (5ml) and the resultant solution was acidified with 2N hydrochloric acid (to pH2). The precipitate was filtered off and purified by h.p.l.c. eluting with 0.05% aqueous trifluoroacetic acid to give the title compound as a white solid (58mg), m.p. 174-176°C. (dec)

Assay Found: C,45.65; H,3.35; N,6.15% $C_{24}H_{22}Br_2ClN_3O_3.0.5CF_3CO_2H$ Requires: C,46.0; H,3.45; N,6.45%

Example (8)

5-[2-[5-[[2-Butyl-4-chloro-5-hydroxymethyl-1H-imidazol-1-yl]methyl]- 1H-indol-2-yl]phenyl]tetrazole

A mixture of the Intermediate (15) (0.22g) and tri-n-butyl tin azide (1.4g) was heated at 155-160°C for 30min under a nitrogen atmosphere. After cooling, 2N sodium hydroxide (5ml) and methanol (3ml) were added to the stirred mixture. After standing overnight the mixture was washed with ether (5x10ml) before being neutralised to pH7 by the addition of 2N hydrochloric acid and washed with further ether (5x10ml). The mixture was made acidic (pH3), methanol (5ml) added and the mixture extracted with ethyl acetate (1x30ml). The organic phase was dried and concentrated in vacuo to afford a brown solid (0.215g). Trituration with ether (25ml) afforded a brown solid (0.135g). Chromatography eluting with ethanol:chloroform (3:5) gave the title compound as a brown solid. (41mg). T.l.c ethyl acetate, Rf 0.15

n.m.r δ ($CH_3OD$) 0.92 (3H,t), 1.37 (2H,m), 1.65 (2H,m), 2.59 (2H,t), 4.47 (2H,s), 5.3 (2H,s), 5.89 (1H,s), 6.81 (1H, dd), 7.03 (1H,d), 7.29 (1H,d), 7.35-7.58 (3H,m), 7.78 (1H,dd).

Example (9)
N-[2-[5-[(2-Butyl-1H-imidazol-1-yl)methyl]-1H-indol-2-yl]phenyl]-1,1,1 -trifluoromethane sulphonamide

Trifluoromethanesulphonic anhydride (0.15ml) was added dropwise to a cooled (-78°C) solution of Intermediate (20) (0.3g) and triethylamine (0.1ml) in dichloromethane (10ml) and the resulting mixture stirred for 4h. Sodium bicarbonate (8%, 10ml) was added and the organic layer separated. The dried organic solution was evaporated in vacuo and the residue purified by chromatography eluting with System E (100:8:1) to give the title compound as an orange gummy solid (150mg)
T.l.c. System E (100:8:1) Rf 0.15
n.m.r δ ($CDCl_3$) 0.86 (3H,t), 1.28 (2H,m), 1.63 (2H,m), 2.67 (2H,t), 5.1 (2H,s), 6.58 (1H,s), 6.72 (1H, d), 6.81 (1H,d), 6.87 (1H,dd), 7.07 (1H,ddd), 7.18-7.3 (2H,m), 7.33 (1H,d), 7.51 (1H,dd), 7.64 (1H,dd).

Example (10)
2-[5-(2-Butyl-1H-imidazol-1-yl)-1-methyl-1H-indol-2-yl]-benzoic acid,2,2,2-trifluoroacetate (1:1:1) salt

A solution of Intermediate (23) (142mg) in methanol (5ml) and aqueous sodium hydroxide (2M, 1ml) was stood at room temperature for 16h. Water (5ml) was added and the mixture concentrated in vacuo - (~5ml). Aqueous hydrochloric acid (2M, 1ml) was added dropwise to neutralise the mixture which was then concentrated in vacuo. The residue was purified by h.p.l.c. to give the title compound as a reddish brown glass (114mg).
n.m.r δ(DMSO) 0.9 (3H,t), 1.35 (2H,m), 1.6 (2H,m), 3.06 (2H,t), 3.5(3H,s), 5.5 (2H,s), 6.42 (1H,s), 7.2 (1H,dd), 7.45-7.75 (7H,m), 7.97 (1H,dd), 12.8 (1H, v.br). HPLC, Dynamax $C_{18}$ 60A, 8μ, 25cm x 41.4i.d.column, mobile phase: acetonitrile/water (containing 0.1% TFA), 9 to 81% acetonitrile over 25 min. Detection λ230nm. Retention time 16.8 min.

Example (11)
2-[[5-(2-Butyl-1H-imidazol-1-yl)methyl-3-formyl]-1H-indol-2-yl]benzoic acid

A solution of Intermediate (26)(100mg) in methanol (5ml) and sodium hydroxide (2N, 2ml) was stirred at room temperature overnight. The solution was concentrated in vacuo and then acidified with hydrochloric acid (2N, 10ml). The precipitate was collected and dried in vacuo to give the title compound as a yellow powder (35mg) m.p. 172-173°C; dec.
n.m.r. δ(DMSO) 0.9 (3H,t), 1.35(2H,m), 1.6(2H,m), 3.06 (2H,t), 7.26 (1H,dd), 7.51 (1H,d) 7.6 (1H,d), 7.61-7.8 (4H,m), 8.06 (1H,dd), 8.16 (1H,d), 9.6 (1H,s), 12.5 (1H,s).

Example (12)
[3-Bromo-5-[(2-butyl-1H-imidazol-1-yl)methyl]-1-methyl-1H-indol -2-yl benzoic acid

A solution of Intermediate (28) (270mg) and aqueous sodium hydroxide (2N, 1ml) in methanol (5ml) was st,ood at room temperature for 16h. The mixture was concentrated in vacuo before adjusting to pH6 with aqueous hydrochloric acid (2M, ca 1ml). The solvent was removed in vacuo and the residue extracted with acetonitrile:water:TFA (9:1:0.005) (5ml), filtered and purified by h.p.l.c. to give a yellow oil. A solution of this oil in dichloromethane (10ml) was washed with water (10ml). The pH of the aqueous phase was adjusted to pH6 with aq. sodium hydroxide (2M), dried ,filtered and evaporated in vacuo to give the title compound as a yellow powder (96.2mg) m.p. 155-160°C.
HPLC - conditions as in Example 10 - Retention Time 18.53 mins.

Example 13
2-[5-[[2-Butyl-4-chloro-5-(hydroxymethyl)-1H-imidazol-1-yl]methyl]-1H-indol-3-yl]benzoic acid

A solution of Intermediate (32) (400mg) in methanol (5ml) and 2N sodium hydroxide solution (3ml) was stirred at room temperature for 3h. The solution was acidified with 2N hydrochloric acid and evaporated to give an orange gum which was purified by H.P.L.C. to give the title compound as a yellow gum (170mg)
Assay Found: C,59.85; H,5.5; N,7.95.
$C_{24}H_{24}ClN_3O_3.0.5C_2HF_3O_2.0.5H_2O$ requires C,59.6; H,5.1; N,8.3%.

HPLC - conditions as in Example 10 - Retention Time = 16.41 mins

Example 14
2-[4-(2-Butyl-1H-imidazol-1-yl)-3-bromo-1H-indol-2-yl]benzoic acid

A solution of Intermediate (37) (0.19g) in methanol (2ml) was treated with 2N sodium hydroxide (1ml). The solution became cloudy, clearing on addition of further methanol (1ml). Stirring at room temperature was continued overnight. The methanol was evaporated and the residue diluted with water (15ml). The suspension was acidified to pH7 and refrigerated for 1h during which time a solid formed which was filtered and dried to give the title compound as a pale yellow solid (0.040g) m.p. 162-165°C
HPLC - conditions as in Example 10 - Retention Time = 16.9 mins

Example 15
1-[[3-Bromo-2-(2-carboxyphenyl)-1H-indol-5-yl]-2-butyl-4-chloro]-1H-imidazole-5-carboxylic acid.

A solution of Intermediate (38) (0.205g) in methanol and aqueous 2M sodium hydroxide was heated at 60°C for 5h. The solvent volume was reduced and the aqueous phase obtained, which was washed with dichloromethane before acidification to pH4. The resulting suspension was extracted with ethyl acetate and the combined organic extracts were dried, filtered and evaporated to give the title compound as an orange solid (0.105g) m.p. 192-194°C.
HPLC Retention time = 22.95 minutes.

Example 16
1-[[3-Bromo-2-[2-(1H-tetrazol-5-yl)phenyl]-1H-indol-5-yl]methyl]-2-butyl-4-chloro-1H-imidazole-5-carboxylic acid

A solution of Intermediate (46) (0.04g) in ethanol (5ml) and 2N sodium hydroxide (2ml) was heated at 65° for 2.5h. After cooling, the solution was acidified with 2N hydrochloric acid and the resultant suspension was filtered and the solid dried to give the title compound (0.024g) as a white solid, m.p. 185°-189°C.
n.m.r. (DMSO) 11.8δ (1H,brs), 7.9δ (1H,m), 7.78 (3H,m), 7.35δ (1H,d), 7.03δ (1H,brs), 6.9δ (1H,dd), 5.68δ - (2H,s), 2.68δ (2H,t), 1.54δ (2H,m), 1.3δ (2H,m), 0.8δ (3H,t)

Example 17
Ethyl 1-[[3-bromo-2-[2-[[(trifluoromethyl)sulphonyl]amino]phenyl]-1H-indol-5-yl]methyl]-2-butyl-4-chloro-1H-imidazole-5-carboxylate

A solution of Intermediate (51) (0.15g) and triethylamine (0.082ml) in dry dichloromethane (10ml) at -80° was treated with a solution of trifluoromethanesulphonylanhydride (0.42ml; 1M) in dry dichloromethane. The mixture was stirred at -80°C to -60°C for 35min, and water (0.5ml) was added. The organic solution was dried and evaporated in vacuo. The residue was purified by column chromatography eluting with a gradient of dichloromethane/methanol (50:1 to 25:1) to give the title compound (0.14g) as an off-white foam, m.p. 101-103°C.
Assay Found: C,47.5; H,4.0; N,8.2;
$C_{26}H_{25}BrClF_3N_4O_4S$ requires C,47.2; H,3.8; N,8.5%

Example 18
1-[[3-Bromo-2-[2-[[(trifluoromethyl)sulphonyl]amino]phenyl]-1H-indol-5-yl]methyl]-2-butyl-4-chloro-1H-imidazole-5-carboxylic acid

A solution of the product of Example 17 (106mg) in a mixture of methanol (6ml) and 2M aqueous sodium hydroxide (2ml) was heated at reflux for 2h. The reaction mixture was evaporated and 2M hydrochloric acid (2ml) was added. The resulting precipitate was crystallized from methanol/water to give the title compound as an off-white solid (90mg), m.p. 196-8°C.
Assay Found: C,45.5; H,3.3; N,8.8;
$C_{24}H_{21}BrClF_3N_4O_4S$ requires C,45.7; H,3.3; N,8.9%

The compounds of the invention are tested in vitro for angiotensin II antagonism. Aortic strips are obtained from male New Zealand white rabbits and prepared for recording isometric contractions in response to cumulative addition of angiotensin II. The potencies of test antagonists are assessed by

measuring their abilities to displace the angiotensin II cumulative concentration response curve. The method used is that of Ackerly et al., Proc. Natl. Acad. Sci., 74(12), pp5725-28 (1977) with the exception that the final composition of the physiological salt solution is as given below in Table 1:

TABLE 1

| Ingredient | Amount (mM) |
|---|---|
| $Na^+$ | 143.4 |
| $K^+$ | 5.9 |
| $Mg2^+$ | 0.6 |
| $Ca2^+$ | 1.3 |
| $Cl^-$ | 124.5 |
| $HPO_4^-$ | 1.2 |
| $SO_4^{2-}$ | 0.6 |
| $HCO_3^-$ | 25.0 |
| glucose | 11.1 |
| indomethacin | 0.005 |
| ascorbic acid | 0.1 |

The tissues are initially challenged with $K^+$ (80mM) and then washed at 0, 5, 10 and 15 minutes after the response to $K^+$ has plateaued. After a further 45 minutes an angiotensin II cumulative response curve is constructed (0.1nM to 0.1μM in 10-fold increments) and the tissues are washed as before. A second, third and fourth angiotensin II cumulative response curve (0.1nM to 0.1μM in 3-fold increments) is then constructed at hourly intervals (15 minutes washing after each curve followed by 45 minutes equilibration). The compounds of the invention (30μM) are tested for angiotensin II antagonism by application 45 minutes before construction of the fourth angiotensin II curve. The third and fourth angiotensin II curves are expressed graphically and a concentration ratio (CR) is calculated by dividing the angiotensin II $EC_{50}$ value obtained in the presence of the test antagonist (i.e. fourth curve) by the angiotensin II $EC_{50}$ value obtained in the absence of the test antagonist (i.e. third curve).

The potency of the test antagonist is expressed as a pKb which is calculated from the equation:

$$pKb = -\log \left[ \frac{CR-1}{[antagonist]} \right]$$

which is a rearrangement of equation 4 described by Furchgott, in Handbook of Exp. Pharmacol., 33, p290 (1972) (eds. Blaschkott and Muscholl).

Compounds of the invention will desirably exhibit a pKb in the range between 5 and 12. Thus we have found that the compounds of the invention inhibit the action of the hormone angiotensin II and are therefore useful in the treatment of conditions in which it is desirable to inhibit angiotensin II activity. In particular, the compounds of the Examples have been tested in the above test and have been found to be active.

There is thus provided as a further aspect of the invention a compound of general formula (I) or a physiologically acceptable salt or a solvate thereof for use in the treatment of conditions associated with excessive or unregulated angiotensin II activity.

In a further or alternative aspect of the invention there is provided a compound of general formula (I) or a physiologically acceptable salt or a solvate thereof for the manufacture of a therapeutic agent for the treatment of conditions associated with excessive or unregulated angiotensin II activity.

There is also provided in a further or alternative aspect of the invention a method for the treatment of conditions associated with excessive or unregulated angiotensin II activity in a mammal including man

comprising administration of an effective amount to a mammal in need of such treatment a compound of general formula (I) or a physiologically acceptable salt or a solvate thereof.

The following examples illustrate pharmaceutical formulations according to the invention. The term "active ingredient" is used herein to represent a compound of formula (I).

Pharmaceutical Example 1

| Oral Tablet A | |
|---|---|
| Active Ingredient | 700mg |
| Sodium starch glycollate | 10mg |
| Microcrystalline cellulose | 50mg |
| Magnesium stearate | 4mg |

Sieve the active ingredient and microcrystalline cellulose through a 40 mesh screen and blend in a appropriate blender. Sieve the sodium starch glycollate and magnesium stearate through a 60 mesh screen, add to the powder blend and blend. until homogeneous. Compress with appropriate punches in an automatic tablet press. The tablets may be coated with a thin polymer coat applied by the film coating techniques well known to those skilled in the art. Pigments may be incorporated in the film coat.

Pharmaceutical Example 2

| Oral Tablet B | |
|---|---|
| Active Ingredient | 500mg |
| Lactose | 100mg |
| Maize Starch | 50mg |
| Polyvinyl pyrrolidone | 3mg |
| Sodium starch glycollate | 10mg |
| Magnesium stearate | 4mg |
| Tablet Weight | 667mg |

Sieve the active ingredient, lactose and maize starch through a 40 mesh screen and blend the powders in a suitable blender. Make an aqueous solution of the polyvinyl pyrrolidone (5 - 10% w/v). Add this solution to the blended powders and mix until granulated; pass the granulate through a 12 mesh screen and dry the granules in a suitable oven or fluid bed dryer. Sieve the remaining components through a 60 mesh screen and blend them with the dried granules. Compress, using appropriate punches, on an automatic tablet press.

The tablets may be coated with a thin polymer coat applied by film coating techniques well known to those skilled in art. Pigments may be incorporated in the film coat.

Pharmaceutical Example 3

| Inhalation Cartridge | |
|---|---|
| Active Ingredient | 1mg |
| Lactose | 24mg |

Blend active ingredient, particle size reduced to a very fine particle size (weight mean diameter ca. 5μm) with the lactose in a suitable powder blender and fill the powder blender into No. 3 hard gelatin capsules.

The contents of the cartridges may be administered using a powder inhaler.

Pharmaceutical Example 4

| Injection Formulation | |
|---|---|
| | % w/v |
| Active ingredient | 1.00 |
| Water for injections B.P. to | 100.00 |

Sodium chloride may be added to adjust the tonicity of the solution and the pH may be adjusted to that of maximum stability and/or to facilitate solution of the active ingredient using dilute acid or alkali or by the addition of suitable buffer salts. Antioxidants and metal chelating salts may also be included.

The solution is prepared, clarified and filled into appropriate sized ampoules sealed by fusion of the glass. The injection is sterilised by heating in an autoclave using one of the acceptable cycles. Alternatively the solution may be sterilised by filtration and filled into sterile ampoules under aseptic conditions. The solution may be packed under an inert atmosphere of nitrogen.

**Claims**

1. A compound of the general formula (I)

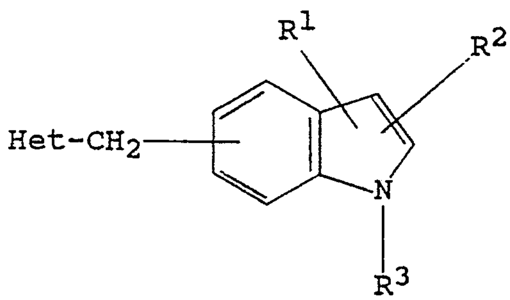

(I)

or a physiologically acceptable salt or solvate thereof wherein
$R^1$ represents a hydrogen atom or a halogen atom or a group selected from $C_{1-6}$alkyl, $C_{2-6}$alkenyl, fluoro$C_{1-6}$alkyl, -CHO, $-CO_2H$ or $-COR^4$;
$R^2$ represents a hydrogen atom or a halogen atom or the group Ar;
$R^3$ represents a hydrogen atom or a group selected from $C_{1-6}$alkyl, $C_{3-6}$alkenyl, $C_{1-6}$alxoxy, $-COR^4$, $-SO_2R^4$ or the group Ar;
$R^4$ represents a group selected from $C_{1-6}$alkyl, $C_{2-6}$alkenyl, $C_{1-6}$alkoxy or the group $-NR^{12}R^{13}$;
Ar represents the group

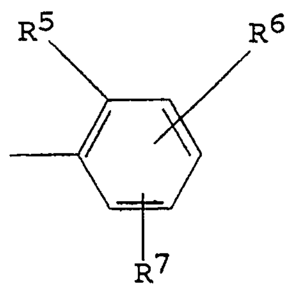

;

$R^5$ represents a group selected from $-CO_2H$, $-NHSO_2CF_3$ or a C-linked tetrazolyl group;
$R^6$ and $R^7$, which may be the same or different, each independently represent a hydrogen atom or a halogen atom or a $C_{1-6}$alkyl group;
Het represents an N-linked imidazolyl group substituted at the 2-position by a $C_{1-6}$alkyl, $C_{2-6}$alkenyl or a $C_{1-6}$alkylthio group, the imidazolyl group optionally being substituted by one or two further substituents selected from a halogen atom or group selected from cyano, nitro, $C_{1-6}$alkyl, $C_{2-6}$alkenyl, fluoro$C_{1-6}$alkyl, $-(CH_2)_mR^8$, $-(CH_2)_nCOR^9$ or $-(CH_2)_pNR^{10}COR^{11}$;
or Het represents an N-linked benzimidazolyl group substituted at the 2-position by a $C_{1-6}$alkyl, $C_{2-6}$alkenyl or a $C_{1-6}$alkylthio group;
$R^8$ represents a hydroxy or $C_{1-6}$alkoxy group;
$R^9$ represents a hydrogen atom or a group selected from hydroxy, $C_{1-6}$alkyl, $C_{1-6}$alkoxy, phenyl, phenoxy or the group $-NR^{12}R^{13}$;
$R^{10}$ represents a hydrogen atom or a $C_{1-6}$alkyl group;
$R^{11}$ represents a hydrogen atom or a group selected from $C_{1-6}$alkyl, $C_{1-6}$alkoxy, phenyl, phenoxy or the group $-NR^{12}R^{13}$;
$R^{12}$ and $R^{13}$, which may be the same or different, each independently represent a hydrogen atom or a $C_{1-4}$alkyl group or $-NR^{12}R^{13}$ forms a saturated heterocyclic ring which has 5 or 6 ring members and may optionally contain in the ring one oxygen atom;
m represents an integer from 1 to 4;
n represents an integer from 0 to 4; and
p represents an integer from 1 to 4;
provided that one of $R^2$ and $R^3$ represents the group, Ar, and that when $R^2$ represents the group Ar, $R^3$ represents a hydrogen atom or a group selected from $C_{1-6}$alkyl, $C_{3-6}$alkenyl, $C_{1-6}$alkoxy, $-COR^4$ or $-SO_2R^4$, and that when $R^3$ represents the group Ar, $R^2$ represents a hydrogen atom or a halogen atom.

2. A compound as claimed in Claim 1 wherein Het is an N-linked imidazolyl group substituted at the 2-position by a $C_{2-5}$alkyl or $C_{3-5}$alkenyl group, for example an n-butyl or but-1-enyl group, and the N-linked imidazolyl group is substituted by one or two further substituents selected from a halogen atom, preferably a chlorine atom, or a group selected from $C_{1-6}$alkyl, $-(CH_2)_mR^8$ or $-(CH_2)_nCOR^9$, especially $-(CH_2)_mR^8$ or $-(CH_2)_nCOR^9$, in which $R^8$ is preferably a hydroxyl group or a methoxy group and m is preferably 1 or 2 and $R^9$ is preferably a hydrogen atom or a hydroxyl, methoxy or ethyoxy group and n is preferably 0, 1 or 2.

3. A compound as claimed in Claim 2 wherein the N-linked imidazolyl group is substituted by a group selected from $-CH_2OH$, -CHO, $-CO_2H$, $-CH_2OCH_3$ or $CO_2CH_2CH_3$.

4. A compound as claimed in any one of Claims 1 to 3 wherein the group Het-$CH_2$- is attached at the 5-position on the indole ring.

5. A compound as claimed in any one of Claims 1 to 4 wherein $R^3$ is a hydrogen atom.

6. A compound as claimed in any one of Claims 1 to 5 wherein $R^2$ represents the group Ar.

7. A compound as claimed in any one of Claims 1 to 6 wherein $R^1$ is attached at the 3-position on the indole ring.

8. A compound as claimed in any one of Claims 1 to 7 wherein $R^1$ represents a hydrogen atom or a halogen atom or a $C_{1-3}$alkyl group, preferably a bromine atom.

9. A compound as claimed in any one of Claims 1 to 8 wherein $R^5$ represents a $-CO_2H$ group.

10. A compound as claimed in any one of Claims 1 to 8 wherein $R^5$ represents a C-linked tetrazolyl group.

11. A compound as claimed in any one Claims 1 to 10 wherein $R^6$ and $R^7$ each independently represent a hydrogen atom.

12. A compound as claimed in Claim 1 or a physiologically acceptable salt or solvent thereof wherein
$R^1$ represents a hydrogen atom or a halogen atom;
$R^2$ represents a halogen atom or the group Ar;
$R^3$ represents a hydrogen atom, a $C_{1-6}$alkyl group or the group Ar;
Ar represents the group

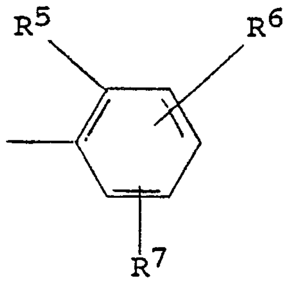

;

$R^5$ represents a group selected from $-CO_2H$, $-NHSO_2CF_3$ or a C-linked tetrazolyl group;
$R^6$ and $R^7$ each independently represent hydrogen atoms;
Het represents an N-linked imidazolyl group substituted at the 2-position by a $C_{1-6}$alkyl group, the imidazolyl group optionally being substituted by one or two further substituents selected from a halogen atom or a group selected from $-(CH_2)_mR^8$, or $-(CH_2)_nCOR^9$;
or Het represents an N-linked benzimidazolyl group substituted at the 2-position by a $C_{1-6}$alkyl group;
$R^8$ represents a hydroxy group;
$R^9$ represents a hydroxy group;
$R^{10}$ represents a hydrogen atom;
$R^{11}$ represents a $C_{1-6}$alkyl group;
m represents an integer from 1 to 4;
n represents an integer from 0 to 4; and
p represents an integer from 1 to 4;
provided that one of $R^2$ and $R^3$ represents the group Ar, and that when $R^2$ represents the group Ar, $R^3$ represents a hydrogen atom or a $C_{1-6}$alkyl group, and that when $R^3$ represents the group Ar, $R^2$ represents a halogen atom.

13. A compound as claimed in Claim 1 selected from:
1-[[3-bromo-2-[2-(1H-tetrazol-5-yl)phenyl]-1H-indol-5-yl]methyl]-2-butyl-4-chloro-1H-imidazole-5-carboxylic acid;
1-[[3-bromo-2-[2-[[(trifluoromethyl)sulphonyl]amino]phenyl]-1H-indol-5-yl]methyl]-2-butyl-4-chloro-1H-imidazole-5-carboxylic acid;
or a physiologically acceptable salt or solvate thereof.

14. A compound as claimed in Claim 1 or a physiologically acceptable salt or solvate thereof wherein
$R^1$ represents a hydrogen atom or a halogen atom or a group selected from $C_{1-6}$alkyl, $C_{2-6}$alkenyl, -CHO, $-CO_2H$ or $-COR^4$;
$R^2$ represents a hydrogen atom or a halogen atom or the group Ar;
$R^3$ represents a hydrogen atom or a group selected from $C_{1-6}$alkyl, $C_{3-6}$alkenyl, $-COR^4$, $-SO_2R^4$ or the group Ar;
$R^4$ represents a group selected from $C_{1-6}$alkyl, $C_{2-6}$alkenyl, $C_{1-6}$alkoxy or the group $-NR^{12}R^{13}$;
Ar represents the group

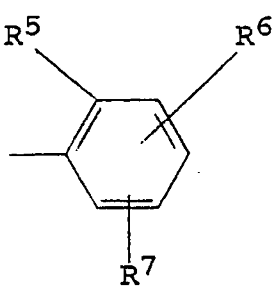

;

$R^5$ represents a group selected from $-CO_2H$, $-NHSO_2CF_3$ or a C-linked tetrazolyl group;
$R^6$ and $R^7$, which may be the same or different, each independently represent a hydrogen atom or a halogen atom or a $C_{1-6}$alkyl group;
Het represents an N-linked imidazolyl group substituted at the 2-position by a $C_{1-6}$alkyl, $C_{2-6}$alkenyl or a $C_{1-6}$alkylthio group, the imidazolyl group optionally being substituted by one or two further substituents

selected from a halogen atom or group selected from cyano, nitro, $C_{1-6}$alkyl, $C_{2-6}$alkenyl, perfluoro$C_{1-3}$alkyl, $-(CH_2)_mR^8$, $-(CH_2)_nCOR^9$ or $-(CH_2)_pNR^{10}COR^{11}$;
or Het represents an N-linked benzimidazolyl group substituted at the 2-position by a $C_{1-6}$alkyl, $C_{2-6}$alkenyl or a $C_{1-6}$alkylthio group;
$R^8$ represents a hydroxy or $C_{1-6}$alkoxy group;
$R^9$ represents a hydrogen atom or a group selected from hydroxy, $C_{1-6}$alkyl, $C_{1-6}$alkoxy, phenyl, phenoxy or the group $-NR^{12}R^{13}$;
$R^{10}$ represents a hydrogen atom or a $C_{1-6}$alkyl group;
$R^{11}$ represents a hydrogen atom or a group selected from $C_{1-6}$alkyl, $C_{1-6}$alkoxy, phenyl, phenoxy or the group $-NR^{12}R^{13}$;
$R^{12}$ and $R^{13}$, which may be the same or different, each independently represent a hydrogen atom or a $C_{1-4}$alkyl group or $-NR^{12}R^{13}$ forms a saturated heterocyclic ring which has 5 or 6 ring members and may optionally contain in the ring one oxygen atom;
m represents an integer from 1 to 4;
n represents an integer from 0 to 4; and
p represents an integer from 1 to 4;
provided that one of $R^2$ and $R^3$ represents the group Ar, and that when $R^2$ represents the group Ar, $R^3$ represents a hydrogen atom or a group selected from $C_{1-6}$alkyl, $C_{3-6}$alkenyl, $-COR^4$ or $-SO_2R^4$, and that when $R^3$ represents the group Ar, $R^2$ represents a hydrogen atom or a halogen atom.

15. A compound as claimed in Claim 1 or a physiologically acceptable salt or solvate thereof wherein
$R^1$ represents a hydrogen atom or a halogen atom or a group selected from $C_{1-6}$alkyl, $C_{2-6}$alkenyl, -CHO, $-CO_2H$ or $-COR^4$;
$R^2$ represents a hydrogen atom or a halogen atom or the group Ar;
$R^3$ represents a hydrogen atom or a group selected from $C_{1-6}$alkyl, $C_{3-6}$alkenyl, $C_{1-6}$alkoxy, $-COR^4$, $-SO_2R^4$ or the group Ar;
$R^4$ represents a group selected from $C_{1-6}$alkyl, $C_{2-6}$alkenyl, $C_{1-6}$alkoxy or the group $-NR^{12}R^{13}$;
Ar represents the group

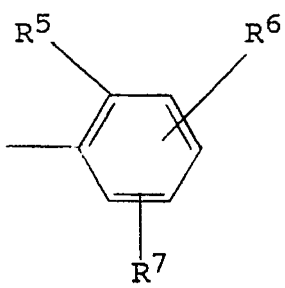

;

$R^5$ represents a group selected from $-CO_2H$, $-NHSO_2CF_3$ or a C-linked tetrazolyl group;
$R^6$ and $R^7$, which may be the same or different, each independently represent a hydrogen atom or a halogen atom or a $C_{1-6}$alkyl group;
Het represents an N-linked imidazolyl group substituted at the 2-position by a $C_{1-6}$alkyl, $C_{2-6}$alkenyl or a $C_{1-6}$alkylthio group, the imidazolyl group optionally being substituted by one or two further substituents selected from a halogen atom or group selected from cyano, nitro, $C_{1-6}$alkyl, $C_{2-6}$alkenyl, fluoro$C_{1-6}$alkyl, $-(CH_2)_mR^8$, $-(CH_2)_nCOR^9$ or $-(CH_2)_pNR^{10}COR^{11}$;
or Het represents an N-linked benzimidazolyl group substituted at the 2-position by a $C_{1-6}$alkyl, $C_{2-6}$alkenyl or a $C_{1-6}$ alkylthio group;
R8 represents a hydroxy or $C_{1-6}$alkoxy group;
$R^9$ represents a hydrogen atom or a group selected from hydroxy, $C_{1-6}$alkyl, $C_{1-6}$alkoxy, phenyl, phenoxy or the group $-NR^{12}R^{13}$;
$R^{10}$ represents a hydrogen atom or a $C_{1-6}$alkyl group;
$R^{11}$ represents a hydrogen atom or a group selected from $C_{1-6}$alkyl, $C_{1-6}$alkoxy, phenyl, phenoxy or the group $-NR^{12}R^{13}$;
$R^{12}$ and $R^{13}$, which may be the same or different, each independently represent a hydrogen atom or a $C_{1-4}$alkyl group or $-NR^{12}R^{13}$ forms a saturated heterocyclic ring which has 5 or 6 ring members and may optionally contain in the ring one oxygen atom;
m represents an integer from 1 to 4;

n represents an integer from 0 to 4; and
p represents an integer from 1 to 4;
provided that one of $R^2$ and $R^3$ represents the group, Ar, and that when $R^2$ represents the group Ar, $R^3$ represents a hydrogen atom or a group selected from $C_{1-6}$alkyl, $C_{3-6}$alkenyl, $C_{1-6}$alkoxy, $-COR^4$ or $-SO_2R^4$, and that when $R^3$ represents the group Ar, $R^2$ represents a hydrogen atom or a halogen atom.

16. A process for the preparation of a compound as claimed in any one of Claims 1 to 15 or a physiologically acceptable salt or solvate thereof which comprises:
(A) reacting an indole of general formula (II)

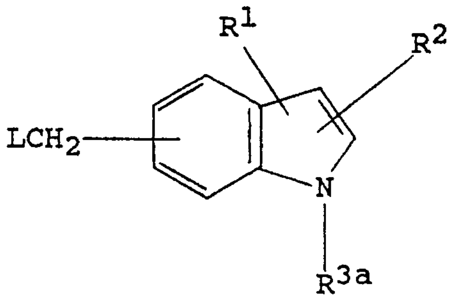

(II)

in which L is a leaving group, with an imidazole of formula (III)

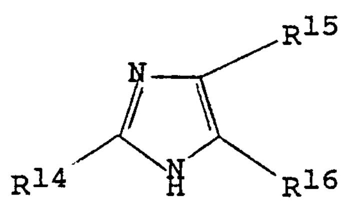

(III)

in which $R^{14}$ represents a group selected from $C_{1-6}$alkyl, $C_{2-6}$alkenyl or $C_{1-6}$alkylthio and $R^{15}$ and $R^{16}$ each independently represent a hydrogen or halogen atom or a group selected from cyano, nitro, $C_{1-6}$alkyl, $C_{2-6}$alkenyl, fluoro$C_{1-6}$alkyl, $-(CH_2)_mR^8$, $-(CH_2)_nCOR^9$, $-(CH_2)_pNR^{10}COR^{11}$ or when taken together with the carbon atoms to which $R^{15}$ and $R^{16}$ are attached, there is formed a fused phenyl ring, followed, if necessary, by removal of any protecting group present; or
(B) deprotecting a protected intermediate of general formula (IV)

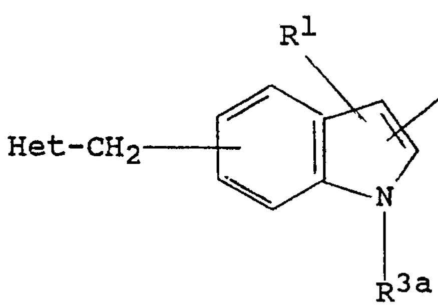

(IV)

in which $R^{3a}$ is a protecting group and/or at least one reactive group is blocked by a protecting group; or
(C) reacting a compound of general formula (Ia)

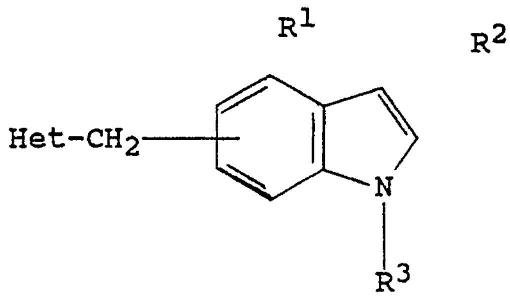

(Ia)

in which $R^5$ in the group Ar as the substituent $R^2$ or $R^3$ represents a nitrile group, with an azide, followed, if necessary, by the removal of any protecting group present; and when the compound of formula (I) is obtained as a mixture of enantiomers optionally resolving the mixture to obtain the desired enantiomer; and/or, if desired, converting the resulting compounds of general formula (I) or a salt thereof into a physiologically acceptable salt or solvate thereof.

17. The process for the preparation of a compound as claimed in any one of Claims 1 to 15 or a physiologically acceptable salt or solvate thereof which comprises:

(A) reacting an indole of general formula (II)

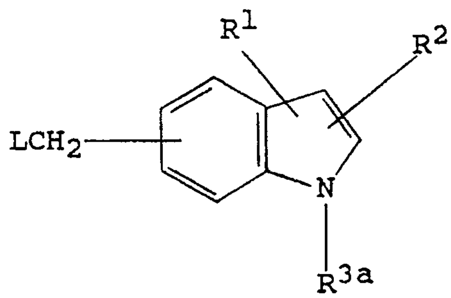

(II)

in which L is a leaving group, with an imidazole of formula (III)

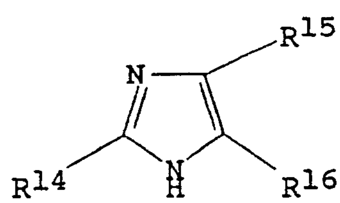

(III)

in which $R^{14}$ represents a group selected from $C_{1-6}$alkyl, $C_{2-6}$alkenyl or $C_{1-6}$alkylthio and $R^{15}$ and $R^{16}$ each independently represent a hydrogen or halogen atom or a group selected from cyano, nitro, $C_{1-6}$alkyl, $C_{2-6}$alkenyl, fluoro$C_{1-6}$alkyl, $-(CH_2)_mR^8$, $-(CH_2)_nCOR^9$, $-(CH_2)_pNR^{10}COR^{11}$ or when taken together with the carbon atoms to which $R^{15}$ and $R^{16}$ are attached, there is formed a fused phenyl ring, followed, if necessary, by removal of any protecting group present; or

(B) deprotecting a protected intermediate of general formula (IV)

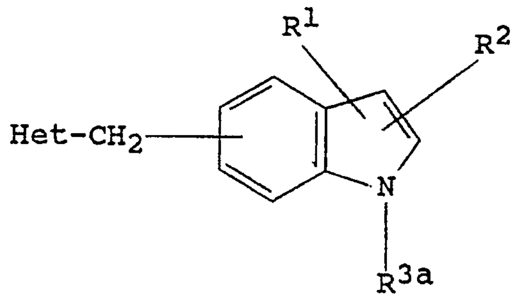

(IV)

in which $R^{3a}$ is a protecting group and/or at least one reactive group is blocked by a protecting group; or (C) reacting a compound of general formula (Ia)

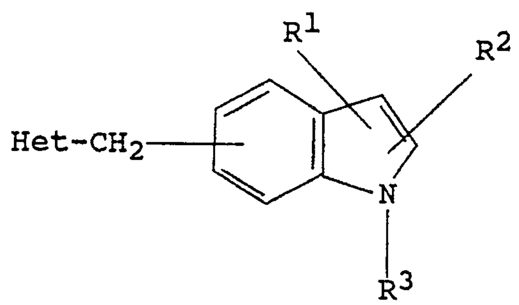

(Ia)

in which $R^5$ in the group Ar as the substituent $R^2$ or $R^3$ represents a nitrile group, with an azide, followed, if necessary, by the removal of any protecting group present; or

(D) converting a compound of general formula (Ib)

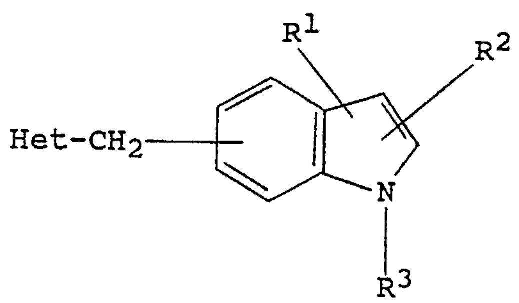

(Ib)

in which $R^5$ in the group Ar as the substituent $R^2$ or $R^3$ represents an amino group, to a compound of general formula (I) in which $R^5$ represents $-NHSO_2CF_3$;

and when the compound of formula (I) is obtained as a mixture of enantiomers optionally resolving the mixture to obtain the desired enantiomers;

and/or, if desired, converting the resulting compounds of general formula (I) or a salt thereof into a physiologically acceptable salt or solvate thereof.

18. Pharmaceutical composition comprising at least one compound of general formula (I) as defined in any one of Claims 1 to 15 or a physiologically acceptable salt or solvate thereof, together with at least one physiologically acceptable carrier or excipient.

19. A compound of general formula (I) as claimed in any one of Claims 1 to 15 or a physiologically acceptable salt or solvate thereof for use in therapy, for example

(i) for use in the treatment or prophylaxis of hypertension; or

(ii) for use in the treatment or prophylaxis of a disease associated with the cognitive disorders, renal failure, hyperaldosteronism, cardiac insufficiency, congestive heart failure, post-myocardial infarction, cerebrovascular disorders, glaucoma and disorders of intracellular homeostasis; or

(iii) for use in the treatment of conditions associated with excessive or unregulated angiotensin II activity.

20. A compound of general formula (II)

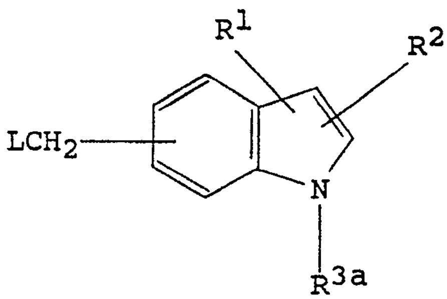

(II)

or an acid addition salt thereof
wherein
$R^1$ and $R^2$ are as defined in Claim 1;
$R^{3a}$ is as defined in $R^3$ in Claim 1 or alternatively represents a nitrogen protecting group; and
L represents a leaving group.

21. A compound of general formula (Ia)

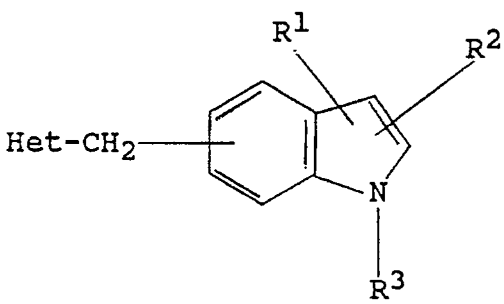

(Ia)

or an acid addition salt thereof
wherein
$R^1$, $R^2$, $R^3$ and Het are as defined in Claim 1 except that in the Group Ar, $R^5$ represents a nitrile group.

22. A compound of general formula (Ib)

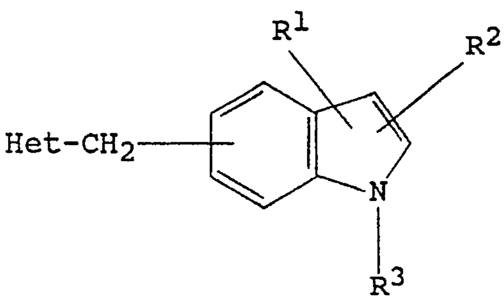

(Ib)

or an acid addition salt thereof wherein $R^1$, $R^2$, $R^3$ and Het are as defined in Claim 1 except that in the Group Ar, $R^5$ represents an amino group.

Claims for the following Contracting States: ES, GR

1. A process for the preparation of a compound of the general formula (I)

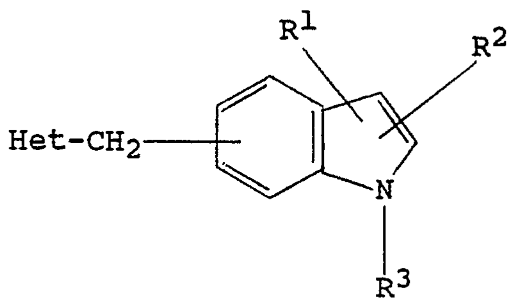

(I)

or a physiologically acceptable salt or solvate thereof wherein

$R^1$ represents a hydrogen atom or a halogen atom or a group selected from $C_{1-6}$alkyl, $C_{2-6}$alkenyl, fluoro$C_{1-6}$alkyl, -CHO, $-CO_2H$ or $-COR^4$;

$R^2$ represents a hydrogen atom or a halogen atom or the group Ar;

$R^3$ represents a hydrogen atom or a group selected from $C_{1-6}$alkyl, $C_{3-6}$alkenyl, $C_{1-6}$alkoxy, $-COR^4$, $-SO_2R^4$ or the group Ar;

$R^4$ represents a group selected from $C_{1-6}$alkyl, $C_{2-6}$alkenyl, $C_{1-6}$alkoxy or the, group $-NR^{12}R^{13}$;

Ar represents the group

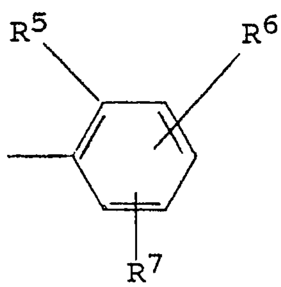

;

$R^5$ represents a group selected from $-CO_2H$, $-NHSO_2CF_3$ or a C-linked tetrazolyl group;

$R^6$ and $R^7$, which may be the same or different, each independently represent a hydrogen atom or a halogen atom or a $C_{1-6}$alkyl group;

Het represents an N-linked imidazolyl group substituted at the 2-position by a $C_{1-6}$alkyl, $C_{2-6}$alkenyl or a $C_{1-6}$alkylthio group, the imidazolyl group optionally being substituted by one or two further substituents selected from a halogen atom or group selected from cyano, nitro, $C_{1-6}$alkyl, $C_{2-6}$alkenyl, fluoro$C_{1-6}$alkyl, $-(CH_2)_mR^8$, $-(CH_2)_nCOR^9$ or $-(CH_2)_pNR^{10}COR^{11}$;

or Het represents an N-linked benzimidazolyl group substituted at the 2-position by a $C_{1-6}$alkyl, $C_{2-6}$alkenyl or a $C_{1-6}$alkylthio group;

$R^8$ represents a hydroxy or $C_{1-6}$alkoxy group;

$R^9$ represents a hydrogen atom or a group selected from hydroxy, $C_{1-6}$alkyl, $C_{1-6}$alkoxy, phenyl, phenoxy or the group $-NR^{12}R^{13}$;

$R^{10}$ represents a hydrogen atom or a $C_{1-6}$alkyl group;

$R^{11}$ represents a hydrogen atom or a group selected from $C_{1-6}$alkyl, $C_{1-6}$alkoxy, phenyl, phenoxy or the group $-NR^{12}R^{13}$;

$R^{12}$ and $R^{13}$, which may be the same or different, each independently represent a hydrogen atom or a $C_{1-4}$alkyl group or $-NR^{12}R^{13}$ forms a saturated heterocyclic ring which has 5 or 6 ring members and may optionally contain in the ring one oxygen atom;

m represents an integer from 1 to 4;

n represents an integer from 0 to 4; and

p represents an integer from 1 to 4;

provided that one of $R^2$ and $R^3$ represents the group Ar, and that when $R^2$ represents the group Ar, $R^3$ represents a hydrogen atom or a group selected from $C_{1-6}$alkyl, $C_{3-6}$alkenyl, $C_{1-6}$alkoxy, $-COR^4$ or $-SO_2R^4$, and that when $R^3$ represents the group Ar, $R^2$ represents a hydrogen atom or a halogen atom;

which comprises:

(A) reacting an indole of general formula (II)

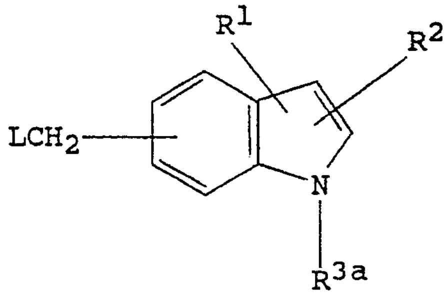

(II)

in which L is a leaving group, with an imidazole of formula (III)

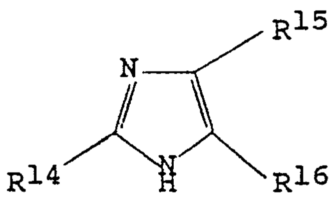

(III)

in which $R^{14}$ represents a group selected from $C_{1-6}$alkyl, $C_{2-6}$alkenyl or $C_{1-6}$alkylthio and $R^{15}$ and $R^{16}$ each independently represent a hydrogen or halogen atom or a group selected from cyano, nitro, $C_{1-6}$alkyl, $C_{2-6}$alkenyl, fluoro$C_{1-6}$alkyl, $-(CH_2)_mR^8$, $-(CH_2)_nCOR^9$, $-(CH_2)_pNR^{10}COR^{11}$ or when taken together with the carbon atoms to which $R^{15}$ and $R^{16}$ are attached, there is formed a fused phenyl ring, followed, if necessary, by removal of any protecting group present; or

(B) deprotecting a protected intermediate of general formula (IV)

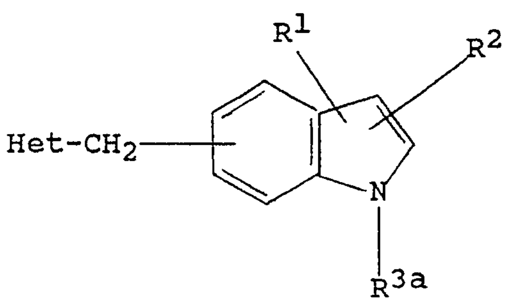

(IV)

in which $R^{3a}$ is a protecting group and/or at least one reactive group is blocked by a protecting group; or

(C) reacting a compound of general formula (Ia)

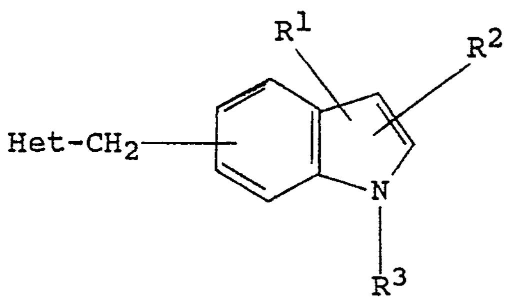

(Ia)

in which $R^5$ in the group Ar as the substituent $R^2$ or $R^3$ represents a nitrile group, with an azide, followed, if

necessary, by the removal of any protecting group present; or
(D) converting a compound of general formula (Ib)

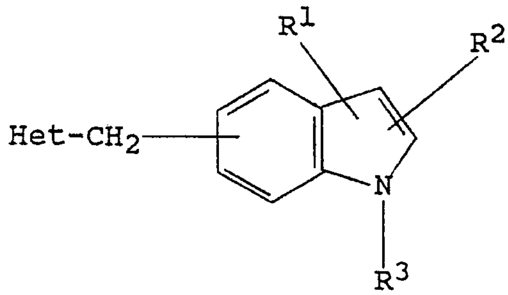

(Ib)

in which $R^5$ in the group Ar as the substituent $R^2$ or $R^3$ represents an amino group, to a compound of general formula (I) in which $R^5$ represents $-NHSO_2CF_3$;
and when the compound of formula (I) is obtained as a mixture of enantiomers optionally resolving the mixture to obtain the desired enantiomer;
and/or, if desired, converting the resulting compounds of general formula (I) or a salt thereof into a physiologically acceptable salt or solvate thereof.
2. A process for the preparation of a compound of the general formula (I) as defined in Claim 1 or a physiologically acceptable salt or solvate thereof which comprises:
(A) reacting an indole of general formula (II)

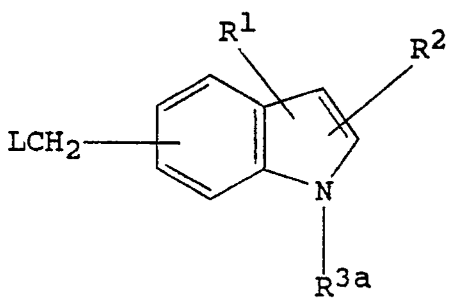

(II)

in which L is a leaving group, with an imidazole of formula (III)

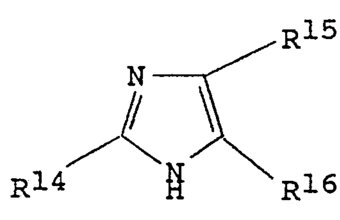

(III)

in which $R^{14}$ represents a group selected from $C_{1-6}$alkyl, $C_{2-6}$alkenyl or $C_{1-6}$alkylthio and $R^{15}$ and $R^{16}$ each independently represent a hydrogen or halogen atom or a group selected from cyano, nitro, $C_{1-6}$alkyl, $C_{2-6}$alkenyl, fluoro$C_{1-6}$alkyl, $-(CH_2)_mR^8$, $-(CH_2)_nCOR^9$, $-(CH_2)_pNR^{10}COR^{11}$ or when taken together with the carbon atoms to which $R^{15}$ and $R^{16}$ are attached, there is formed a fused phenyl ring, followed, if necessary, by removal of any protecting group present; or
(B) deprotecting a protected intermediate of general formula (IV)

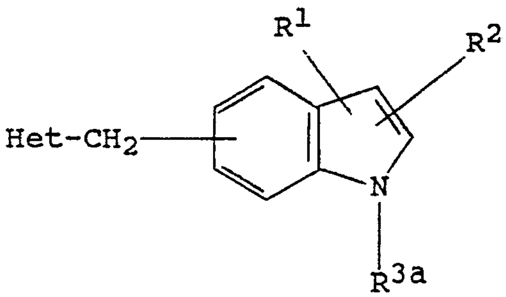

(IV)

in which $R^{3a}$ is a protecting group and/or at least one reactive group is blocked by a protecting group; or (C) reacting a compound of general formula (Ia)

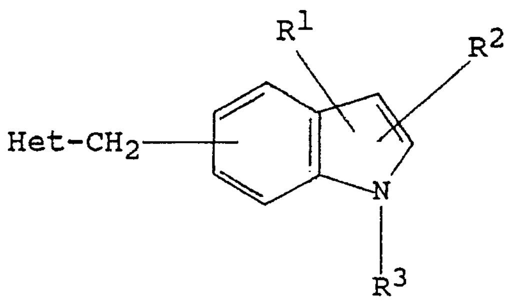

(Ia)

in which $R^5$ in the group Ar as the substituent $R^2$ or $R^3$ represents a nitrile group, with an azide, followed, if necessary, by the removal of any protecting group present;
and when the compound of formula (I) is obtained as a mixture of enantiomers optionally resolving the mixture to obtain the desired entantiomer;
and/or if desired, converting the resulting compounds of general formula (I) or a salt thereof into a physiologically acceptable salt or solvate thereof.

3. A process as claimed in Claim 1 or 2 for the preparation of a compound wherein Het is an N-linked imidazolyl group substituted at the 2-position by a $C_{2-5}$alkyl or $C_{3-5}$alkenyl group, for example an n-butyl or but-1-enyl group, and the N-linked imidazolyl group is substituted by one or two further substituents selected from a halogen atom, preferably a chlorine atom, or a group selected from $C_{1-6}$alkyl, $-(CH_2)_mR^8$ or $-(CH_2)_nCOR^9$, especially $-(CH_2)_mR^8$ or $-(CH_2)_nCOR^9$, in which $R^8$ is preferably a hydroxyl group or a methoxy group and m is preferably 1 or 2 and $R^9$ is preferably a hydrogen atom or a hydroxyl, methoxy or ethoxy group and n is preferably 0, 1 or 2.

4. A process as claimed in Claim 3 for the preparation of a compound wherein the N-linked imidazolyl group is substituted by a group selected from $-CH_2OH$, $-CHO$, $-CO_2H$, $-CH_2OCH_3$ or $CO_2CH_2CH_3$.

5. A process as claimed in any one of Claims 1 to 4 for the preparation of a compound wherein the group Het-$CH_2$- is attached at the 5-position on the indole ring.

6. A process as claimed in any one of Claims 1 to 5 for the preparation of a compound wherein $R^3$ is a hydrogen atom.

7. A process as claimed in any one of Claims 1 to 6 for the preparation of a compound wherein $R^2$ represents the group Ar.

8. A process as claimed in any one of Claims 1 to 7 for the preparation of a compound wherein $R^1$ is attached at the 3-position on the indole ring.

9. A process as claimed in any one of Claims 1 to 8 for the preparation of a compound wherein $R^1$ represents a hydrogen atom or a halogen atom or a $C_{1-3}$alkyl group, preferably a bromine atom.

10. A process as claimed in any one of Claims 1 to 9 for the preparation of a compound wherein $R^5$ represents a $-CO_2H$ group.

11. A process as claimed in any one of Claims 1 to 9 for the preparation of a compound wherein $R^5$ represents a C-linked tetrazolyl group.

12. A process as claimed in any one Claims 1 to 11 for the preparation of a compound wherein $R^6$ and $R^7$ each independently represent a hydrogen atom.

13. A process as claimed in Claim 1 or 2 for the preparation of a compound of the general formula (I) or a physiologically acceptable salt or solvate thereof wherein
$R^1$ represents a hydrogen atom or a halogen atom;
$R^2$ represents a halogen atom or the group Ar;
$R^3$ represents a hydrogen atom, a $C_{1-6}$alkyl group or the group Ar;
Ar represents the group

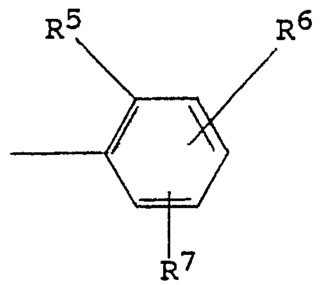

;

$R^5$ represents a group selected from $-CO_2H$, $-NHSO_2CF_3$ or a C-linked tetrazolyl group;
$R^6$ and $R^7$ each independently represent hydrogen atoms;
Het represents an N-linked imidazolyl group substituted at the 2-position by a $C_{1-6}$alkyl group, the imidazolyl group optionally being substituted by one or two further substituents selected from a halogen atom or a group selected from $-(CH_2)_mR^8$, or $-(CH_2)_nCOR^9$;
or Het represents an N-linked benzimidazolyl group substituted at the 2-position by a $C_{1-6}$alkyl group;
$R^8$ represents a hydroxy group;
$R^9$ represents a hydroxy group;
$R^{10}$ represents a hydrogen atom;
$R^{11}$ represents a $C_{1-6}$alkyl group;
m represents an integer from 1 to 4;
n represents an integer from 0 to 4; and
p represents an integer from 1 to 4;
provided that one of $R^2$ and $R^3$ represents the group Ar, and that when $R^2$ represents the group Ar, $R^3$ represents a hydrogen atom or a $C_{1-6}$alkyl group, and that when $R^3$ represents the group Ar, $R^2$ represents a halogen atom.

14. A compound as claimed in Claim 1 or 2 from the preparation of a compound selected from:
1-[[3-bromo-2-[2-(1H-tetrazol-5-yl)phenyl]-1H-indol-5-yl]methyl]-2-butyl-4-chloro-1H-imidazole-5-carboxylic acid;
1-[[3-bromo-2-[2-[[(trifluoromethyl)sulphonyl]amino]phenyl]-1H-indol-5-yl]methyl]-2-butyl-4-chloro-1H-imidazole-5-carboxylic acid;
or a physiologically acceptable salt or solvate thereof.

15. A compound as claimed in Claim 1 or 2 for the preparation of a compound of the general formula (I) or a physiologically acceptable salt or solvate thereof wherein
$R^1$ represents a hydrogen atom or a halogen atom or a group selected from $C_{1-6}$alkyl, $C_{2-6}$alkenyl, -CHO, $-CO_2H$ or $-COR^4$;
$R^2$ represents a hydrogen atom or a group selected from group Ar;
$R^3$ represents a hydrogen atom or a group selected from $C_{1-6}$alkyl, $C_{3-6}$alkenyl, $-COR^4$, $-SO_2R^4$ or the group Ar;
$R^4$ represents a group selected from $C_{1-6}$alkyl, $C_{2-6}$alkenyl, $C_{1-6}$alkoxy or the group $-NR^{12}R^{13}$;
Ar represents the group

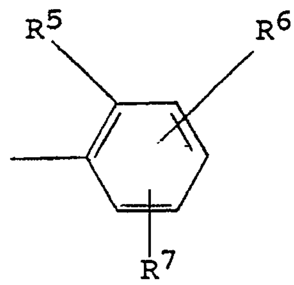

;

$R^5$ represents a group selected from $-CO_2H$, $-NHSO_2CF_3$ or a C-linked tetrazolyl group;
$R^6$ and $R^7$, which may be the same or different, each independently represent a hydrogen atom or a halogen atom or a $C_{1-6}$alkyl group;
Het represents an N-linked imidazolyl group substituted at the 2-position by a $C_{1-6}$alkyl, $C_{2-6}$alkenyl or a $C_{1-6}$alkylthio group, the imidazolyl group optionally being substituted by one or two further substituents selected from a halogen atom or group selected from cyano, nitro, $C_{1-6}$alkyl, $C_{2-6}$alkenyl, perfluoro$C_{1-3}$alkyl, $-(CH_2)_mR^8$, $-(CH_2)_nCOR^9$ or $-(CH_2)_pNR^{10}COR^{11}$;
or Het represents an N-linked benzimidazolyl group substituted at the 2-position by a $C_{1-6}$alkyl, $C_{2-6}$alkenyl or a $C_{1-6}$alkylthio group;
$R^8$ represents a hydroxy or $C_{1-6}$alkoxy group;
$R^9$ represents a hydrogen atom or a group selected from hydroxy, $C_{1-6}$alkyl, $C_{1-6}$alkoxy, phenyl, phenoxy or the group $-NR^{12}R^{13}$;
$R^{10}$ represents a hydrogen atom or a $C_{1-6}$alkyl group;
$R^{11}$ represents a hydrogen atom or a group selected from $C_{1-6}$alkyl, $C_{1-6}$alkoxy, phenyl, phenoxy or the group $-NR^{12}R^{13}$;
$R^{12}$ and $R^{13}$, which may be the same or different, each independently represent a hydrogen atom or a $C_{1-4}$alkyl group or $-NR^{12}R^{13}$ forms a saturated heterocyclic ring which has 5 or 6 ring members and may optionally contain in the ring one oxygen atom;
m represents an integer from 1 to 4;
n represents an integer from 0 to 4; and
p represents an integer from 1 to 4;
provided that one of $R^2$ and $R^3$ represents the group Ar, and that when $R^2$ represents the group Ar, $R^3$ represents a hydrogen atom or a group selected from $C_{1-6}$alkyl, $C_{3-6}$alkenyl, $-COR^4$ or $-SO_2R^4$, and that when $R^3$ represents the group Ar, $R^2$ represents a hydrogen atom or a halogen atom.

16. A process as claimed in Claim 1 or 2 for the preparation of a compound of the general formula (I) or a physiologically acceptable salt or solvate thereof wherein
$R^1$ represents a hydrogen atom or a halogen atom or a group selected from $C_{1-6}$alkyl, $C_{2-6}$alkenyl, $-CHO$, $-CO_2H$ or $-COR^4$;
$R^2$ represents a hydrogen atom or a halogen atom or the group Ar;
$R^3$ represents a hydrogen atom or a group selected from $C_{1-6}$alkyl, $C_{3-6}$alkenyl, $C_{1-6}$alkoxy, $-COR^4$, $-SO_2R^4$ or the group Ar;
$R^4$ represents a group selected from $C_{1-6}$alkyl, $C_{2-6}$alkenyl, $C_{1-6}$alkoxy or the group $-NR^{12}R^{13}$;
Ar represents the group

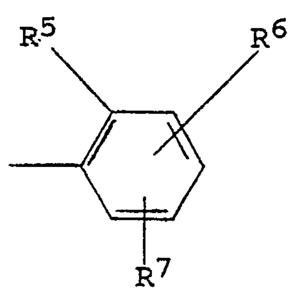

;

$R^5$ represents a group selected from $-CO_2H$, $-NHSO_2CF_3$ or a C-linked tetrazolyl group;
$R^6$ and $R^7$, which may be the same or different, each independently represent a hydrogen atom or a

halogen atom or a $C_{1-6}$alkyl group;

Het represents an N-linked imidazolyl group substituted at the 2-position by a $C_{1-6}$alkyl, $C_{2-6}$alkenyl or a $C_{1-6}$alkylthio group, the imidazolyl group optionally being substituted by one or two further substituents selected from a halogen atom or group selected from cyano, nitro, $C_{1-6}$alkyl, $C_{2-6}$alkenyl, fluoro$C_{1-6}$alkyl, $-(CH_2)_mR^8$, $-(CH_2)_nCOR^9$ or $-(CH_2)_pNR^{10}COR^{11}$;

or Het represents an N-linked benzimidazolyl group substituted at the 2-position by a $C_{1-6}$alkyl, $C_{2-6}$alkenyl or a $C_{1-6}$ alkylthio group;

R8 represents a hydroxy or $C_{1-6}$alkoxy group;

$R^9$ represents a hydrogen atom or a group selected from hydroxy, $C_{1-6}$alkyl, $C_{1-6}$alkoxy, phenyl, phenoxy or the group $-NR^{12}R^{13}$;

$R^{10}$ represents a hydrogen atom or a $C_{1-6}$alkyl group;

$R^{11}$ represents a hydrogen atom or a group selected from $C_{1-6}$alkyl, $C_{1-6}$alkoxy, phenyl, phenoxy or the group $-NR^{12}R^{13}$;

$R^{12}$ and $R^{13}$, which may be the same or different, each independently represent a hydrogen atom or a $C_{1-4}$alkyl group or $-NR^{12}R^{13}$ forms a saturated heterocyclic ring which has 5 or 6 ring members and may optionally contain in the ring one oxygen atom;

m represents an integer from 1 to 4;

n represents an integer from 0 to 4; and

p represents an integer from 1 to 4;

provided that one of $R^2$ and $R^3$ represents the group Ar, and that when $R^2$ represents the group Ar, $R^3$ represents a hydrogen atom or a group selected from $C_{1-6}$alkyl, $C_{3-6}$alkenyl, $C_{1-6}$alkoxy, $-COR^4$ or $-SO_2R^4$, and that when $R^3$ represents the group Ar, $R^2$ represents a hydrogen atom or a halogen atom.